# EUROPEAN PATENT SPECIFICATION

(11) **EP 4 021 295 B1**
(45) Date of publication and mention of the grant of the patent: **15.10.2025**
(21) Application number: 20858333.6
(22) Date of filing: 27.08.2020
(51) Int. Cl.: A61B 5/103, A61B 6/00, A61N 5/00, G06T 7/38, A61B 5/091, A61B 5/00, A61B 5/026, A61B 5/055, A61B 5/11, A61B 5/113, A61B 6/03, A61B 6/08, A61B 6/46, A61B 6/50, A61N 5/10, G06T 7/00, G06T 7/20

(54) **SYSTEM AND APPARATUS FOR ASSESSING AN EFFECT OF A MEDICAL TREATMENT ON ORGAN FUNCTION**
SYSTEM UND VORRICHTUNG ZUR BEURTEILUNG DER WIRKUNG EINER MEDIZINISCHEN BEHANDLUNG AUF ORGANFUNKTIONEN
SYSTÈME ET APPAREIL POUR ÉVALUER UN EFFET D'UN TRAITEMENT MÉDICAL SUR UNE FONCTION D'UN ORGANE

(30) Priority: 27.08.2019 US 201962892485 P
(43) Date of publication of application: 06.07.2022
(73) Proprietor: 4DMedical Limited, Melbourne, VIC 3004 (AU)
(72) Inventor: FOURAS, Andreas, California, 91367 (US)
(74) Representative: Grünecker Patent- und Rechtsanwälte PartG mbB
(86) International application number: PCT/AU2020/050900
(87) International publication number: WO 2021/035304

(56) References cited:
- WO-A1-2008/154741
- WO-A1-2018/157191
- WO-A2-2006/009751
- US-A1- 2005 065 421
- US-A1- 2009 005 693
- US-A1- 2018 260 955

## Description

### FIELD OF THE INVENTION

The present disclosure relates generally to methods, systems, and apparatus for assessing an effect of a medical treatment on an organ, and more particularly, to methods and systems for assessing overall organ function and regional organ function after a treatment, and determining if a treatment has either positively altered, negatively altered, or had no effect on organ function.

### BACKGROUND

Numerous types of treatments for diseased organs exist. For example, a diseased lung may be treated non-invasively with radiation therapy, proton therapy, and antibody therapy, or invasively through surgical dissection of tumors, ablation of tumors, stent placement, valve placement, and glue application to seal lung punctures. Monitoring changes in organ function resulting from these treatments is complex and the ability to assess the effectiveness of these treatments is limited. Even in its simplest set of possibilities: 1) diseased tissue of an organ can be worsening in function due to disease progression, or can be improving in function due to treatment, and 2) healthy tissue could be remaining healthy, becoming effected by disease, or could be responding to toxic or negative "off target" effects of treatment.

Considering these possibilities further in the context of a lung having a cancerous tumor that is being treated by radiation. A radiation treatment plan may prescribe the delivery of a dose of radiation to the tumor at a target location in the body. As used herein, a "dose" refers to a specified amount of treatment, e.g., radiation level/rate, delivered during one treatment session. Radiation delivery, however, is not limited to the target tumor, and lung tissue surrounding or otherwise in the vicinity of the target tumor is also exposed to radiation, although typically at a lower dose. Furthermore, tissue of other organs near the target tumor may also be exposed to and affected by radiation. For example, cardiac tissue adjacent the lung may be exposed to and affected by radiation treatment of the lung.

It is therefore desirable to provide the ability to assess the effect of a medical treatment on organ function. It is further desirable to provide this capability on a regional basis of an organ, i.e., to measure tissue function at different regions of an organ, and to compare or correlate these regional measurements, on a region by region basis, with the treatment. It is also desirable to provide the capability to assess the effect of a treatment on adjacent organs. The concepts disclosed below address these desires and others.

WO 2018 / 157 191 A1 relates to a method of scanning for vascular ill health using a data set from an in vivo scan, the method including the steps of: (1) extracting blood vessel location data and blood vessel size data from the scan data set; (2) selecting a region in the extracted vessel location data; and (3) comparing the size data in the selected region to size data in a corresponding region of a normative data set to determine vascular health.

US 2009 / 0 005 693 A1 teaches methods for analyzing representations of one or more in situ structures in the body of a subject to glean information about the health of the subject. In addition, methods are disclosed for diagnosing, staging, grading, and monitoring diseases. Methods also are disclosed for targeting treatments and screening, validating therapies based on the analysis of in situ patters, and monitoring the effectiveness of therapies.

### SUMMARY

The present disclosure relates to a non-transitory computer readable storage medium having a computer program stored therein, that when executed by a processor of a computer, causes the computer to execute steps directed to assessing an effect of a treatment on an organ, the steps comprising: acquiring a first measurement for each of a plurality of regions of the organ; and acquiring, after acquisition of the first measurements, a second measurement for each of the plurality of regions of the organ. The method further includes obtaining a regional change measurement for each of the plurality of regions of the organ based on the first measurement and the second measurement of the region; and determining a treatment effect based the plurality of regional change measurements and regional treatment information of the treatment delivered to the organ. The treatment effect is determined by: mapping each of the plurality of regional change measurements with a corresponding regional treatment information of the treatment delivered to the organ to generate a mapping; and deriving the treatment effect from the mapping.

In an embodiment, acquiring either of a first measurement for each of a plurality of regions of the organ or a second measurement for each of a plurality of regions of the organ comprises: obtaining a time series of two-dimensional (2D) images of the organ; and processing the time series of 2D images to obtain a motion measurement for each of the plurality of regions. Obtaining a time series of 2D images of the organ may comprises receiving the time series of 2D images from an imaging apparatus or an image source. Additionally or alternatively, obtaining a time series of 2D images of the organ comprises capturing a plurality of time series of 2D images of the organ, each from a different angle relative to the organ. In this instance, the plurality of time series of 2D images of the organ are captured from no more than ten different angles. The plurality of time series of 2D images may be captured simultaneously.

When processing the time series of 2D images the method, in one embodiment, may comprise cross-correlating the 2D images of the organ. This may comprise reconstructing motion measurements for each of the plurality of regions of the organ from the time series of 2D images of the organ, wherein the plurality of regions of the organ comprise tissue of the organ and the motion measurements represent motion of the tissue. Alternatively or additionally, reconstructing motion measurements could comprise reconstructing 3D motion measurements without first reconstructing a 3D image. Processing the time series of 2D images may also further comprise, for each of the plurality of regions of the organ, deriving a volume measurement from one or more motion measurements associated with that region.

In an embodiment disclosed herein, each of the plurality of first measurements is acquired before the treatment, and each of the plurality of second measurements is acquired either during the treatment or after the treatment; or alternatively each of the plurality of first measurements is acquired during the treatment, and each of the plurality of second measurements is acquired after the treatment.

The first measurement and the second measurement of the method may be one of: displacement measurements, velocity measurements, ventilation measurements, perfusion measurements, ventilation/perfusion (V/Q) ratio measurements, or any measurements that may be derived from any of the foregoing measurements.

The step of obtaining a regional change measurement for a region may comprise comparing the first measurement of the region to the second measurement of the region.

Deriving the treatment effect from the mapping could comprise fitting a line through a plot of regional change measurements as a function of regional treatment information. A further step may comprise determining whether the treatment has altered regional organ function based on the treatment effect. Alternatively or additionally, organ function may be assessed based on the treatment effect. The treatment effect may be indicative of one of: a) no change in organ function; b) change in organ function linked to treatment; or c) change in organ function not linked to treatment.

In an embodiment of the method of assessing an effect of a treatment on an organ, the treatment is a non-uniform treatment characterized by varying levels of treatment delivery throughout the organ. The treatment may comprise at least one of radiation therapy, proton therapy, antibody therapy, surgery, valve placement, heat/ablation, or glue. In a particular embodiment, the treatment is a radiation therapy treatment, and the regional treatment information is a dose map comprising a radiation level for each of the plurality of regions of the organ.

The method of assessing an effect of a treatment on an organ, could further comprise either of: prior to obtaining a regional change measurement for each of the plurality of regions of the organ, associating the plurality of first measurements and the plurality of second measurements with a fluid flow structure of the organ; or prior to determining a treatment effect, associating the plurality of regional change measurements with a fluid flow structure of the organ.

In an embodiment, the organ corresponds to a lung and the fluid flow structure corresponds to one of an airway tree of the lung or a vascular tree of the lung, or the organ corresponds to a heart and the fluid flow structure corresponds to a vascular structure of the heart.

The present disclosure also relates to a system for assessing an effect of a treatment on an organ. The system includes a measurement acquisition module, a measurement change module, and a treatment effect module. The measurement acquisition module is configured to acquire a first measurement for each of a plurality of regions of the organ, and acquire, after acquisition of the first measurements, a second measurement for each of the plurality of regions of the organ. The measurement change module is configured to obtain a regional change measurement for each of the plurality of regions of the organ based on the first measurement and the second measurement of the region. The treatment effect module is configured to determine a treatment effect based the plurality of regional change measurements and regional treatment information of the treatment delivered to the organ. The treatment effect module determines a treatment effect by being configured to: map each of the plurality of regional change measurements with a corresponding regional treatment information of the treatment delivered to the organ to generate a mapping; and derive the treatment effect from the mapping.

In one embodiment of the system the measurement acquisition module acquires either of a first measurement for each of a plurality of regions of the organ or a second measurement for each of a plurality of regions of the organ by being configured to: obtain a time series of two-dimensional (2D) images of the organ; and process the time series of 2D images to obtain a motion measurement for each of the plurality of regions. The measurement acquisition module may obtain a time series of 2D images of the organ by being further configured to receive the time series of 2D images from an imaging apparatus or an image source. Alternatively or additionally, the measurement acquisition module obtains a time series of 2D images of the organ by being configured to capture a plurality of time series of 2D images of the organ, each from a different angle relative to the organ. Preferably, the plurality of time series of 2D images of the organ are captured from no more than ten different angles. The plurality of time series of 2D images can be captured simultaneously.

The measurement acquisition module may be capable of processing the time series of 2D images by being further configured to cross-correlate the 2D images of the organ. This could be achieved by reconstructing motion measurements for each of the plurality of regions of the organ from the time series of 2D images of the organ, and/or the measurement acquisition module reconstructs motion measurements by being configured to reconstruct 3D motion measurements without first reconstructing a 3D image.

In one embodiment, the measurement acquisition module processes the time series of 2D images by being configured to, for each of the plurality of regions of the organ, derive a volume measurement from one or more motion measurements associated with that region.

In an embodiment of the system for assessing an effect of a treatment on an organ, the measurement acquisition module is configured to: acquire each of the plurality of first measurements before the treatment, and each of the plurality of second measurements is acquired either during the treatment or after the treatment; or acquire each of the plurality of first measurements during the treatment, and each of the plurality of second measurements is acquired after the treatment.

In the system, the first measurement and the second measurement may be one of: displacement measurements, velocity measurements, ventilation measurements, perfusion measurements, ventilation/perfusion (V/Q) ratio measurements, or any measurements that may be derived from any of the foregoing measurements.

In the system, the measurement change module in one embodiment may obtain a regional change measurement for a region by being configured to compare the first measurement of the region to the second measurement of the region.

The treatment effect module could derive the treatment effect from the mapping by being configured to fit a line through a plot of regional change measurements as a function of regional treatment information. Furthermore or alternatively, the treatment effect module could be configured to: determine whether the treatment has altered regional organ function based on the treatment effect; and/or assess organ function based on the treatment effect.

In an embodiment of the system, the treatment effect is indicative of one of: a) no change in organ function; b) change in organ function linked to treatment; or c) change in organ function not linked to treatment.

In an embodiment of the system, the treatment is a non-uniform treatment characterized by varying levels of treatment delivery throughout the organ. The treatment could comprise at least one of radiation therapy, proton therapy, antibody therapy, surgery, valve placement, heat/ablation, or glue. When the treatment is a radiation therapy treatment, the regional treatment information is a dose map comprising a radiation level for each of the plurality of regions of the organ.

In one embodiment of the system for assessing an effect of a treatment on an organ, the measurement acquisition module is further configured to either of: associate the plurality of first measurements and the plurality of second measurements to a fluid flow structure of the organ, prior to obtaining a regional change measurement for each of the plurality of regions of the organ; or associate the plurality of regional change measurements to a fluid flow structure of the organ, prior to determining a treatment effect.

In use of the system, the organ may correspond to a lung and the fluid flow structure corresponds to one of an airway tree of the lung or a vascular tree of the lung, or the organ may correspond to a heart and the fluid flow structure corresponds to a vascular structure of the heart.

The present disclosure further relates to an apparatus for assessing an effect of a treatment on an organ. The apparatus includes an interface, a memory, and a processor coupled to the interface and the memory. The processor is configured to execute instructions in the memory to cause the apparatus to: acquire a first measurement for each of a plurality of regions of the organ; acquire, after acquisition of the first measurements, a second measurement for each of the plurality of regions of the organ; obtain a regional change measurement for each of the plurality of regions of the organ based on the first measurement and the second measurement of the region; and determine a treatment effect based the plurality of regional change measurements and regional treatment information of the treatment delivered to the organ. The processor causes the apparatus to determine a treatment effect by being configured to execute instructions in the memory to cause the apparatus to: receive regional treatment information from a treatment apparatus or treatment information source; map each of the plurality of regional change measurements with a corresponding regional treatment information of the treatment delivered to the organ to generate a mapping; and derive the treatment effect from the mapping.

In an embodiment of the apparatus, the processor causes the apparatus to acquire either of a first measurement for each of a plurality of regions of the organ or a second measurement for each of a plurality of regions of the organ by being configured to execute instructions in the memory to cause the apparatus to: obtain a time series of two-dimensional (2D) images of the organ from an imaging apparatus or image source; and process the time series of 2D images to obtain a motion measurement for each of the plurality of regions. The processor causes the apparatus to process the time series of 2D images by being further configured to execute instructions in the memory to cause the apparatus to cross-correlate the 2D images of the organ; and/or to process the time series of 2D images by being configured to execute instructions in the memory to cause the apparatus to reconstruct motion measurements for each of the plurality of regions of the organ from the time series of 2D images of the organ.

The processor may cause the apparatus to reconstruct motion measurements by being configured to execute instructions in the memory to cause the apparatus to reconstruct 3D motion measurements without first reconstructing a 3D image. Additionally or alternatively, the processor causes the apparatus to process the time series of 2D images by being configured to execute instructions in the memory to cause the apparatus to, for each of the plurality of regions of the organ, derive a volume measurement from one or more motion measurements associated with that region.

In an embodiment of the apparatus for assessing an effect of a treatment on an organ, the first measurement and the second measurement are one of: displacement measurements, velocity measurements, ventilation measurements, perfusion measurements, ventilation/perfusion (V/Q) ratio measurements, or any measurements that may be derived from any of the foregoing measurements.

In an embodiment, the processor causes the apparatus to obtain a regional change measurement for a region by being configured to execute instructions in the memory to cause the apparatus to compare the first measurement of the region to the second measurement of the region.

The processor may cause the apparatus to derive the treatment effect from the mapping by being configured to execute instructions in the memory to cause the apparatus fit a line through a plot of regional change measurements as a function of regional treatment information.

Alternatively or additionally in the apparatus disclosed, the processor is further configured to execute instructions in the memory to cause the apparatus: determine whether the treatment has altered regional organ function based on the treatment effect. Alternatively or additionally, the processor is further configured to execute instructions in the memory to cause the apparatus: assess organ function based on the treatment effect.

In the apparatus, the treatment effect is preferably indicative of one of: a) no change in organ function; b) change in organ function linked to treatment; or c) change in organ function not linked to treatment.

In one embodiment of the apparatus, the treatment is a non-uniform treatment characterized by varying levels of treatment delivery throughout the organ. The treatment may comprise at least one of radiation therapy, proton therapy, antibody therapy, surgery, valve placement, heat/ablation, or glue. When the treatment is a radiation therapy treatment, the regional treatment information is a dose map comprising a radiation level for each of the plurality of regions of the organ.

In the apparatus for assessing an effect of a treatment on an organ, the processor may be further configured to execute instructions in the memory to cause the apparatus to either of: associate the plurality of first measurements and the plurality of second measurements to a fluid flow structure of the organ, prior to obtaining a regional change measurement for each of the plurality of regions of the organ; or associate the plurality of regional change measurements to a fluid flow structure of the organ, prior to determining a treatment effect.

In use of the apparatus, the organ may correspond to a lung and the fluid flow structure corresponds to one of an airway tree of the lung or a vascular tree of the lung, or the organ may correspond to a heart and the fluid flow structure corresponds to a vascular structure of the heart.

The present disclosure also relates to a non-transitory computer readable storage medium having a computer program stored therein, that when executed by a processor of a computer, causes the computer to execute steps directed to assessing an effect of a treatment on an organ. The steps include acquiring a first measurement for each of a plurality of regions of an organ; acquiring, after acquisition of the first measurements, a second measurement for each of the plurality of regions of the organ; obtaining a regional change measurement for each of the plurality of regions of the organ based on the first measurement and the second measurement of the region; and determining a treatment effect based the plurality of regional change measurements and regional treatment information of the treatment delivered to the organ.

It is understood that other aspects of apparatuses and methods will become readily apparent to those skilled in the art from the following detailed description, wherein various aspects of apparatuses and methods are shown and described by way of illustration. As will be realized, these aspects may be implemented in other and different forms and its several details are capable of modification in various other respects. Accordingly, the drawings and detailed description are to be regarded as illustrative in nature and not as restrictive.

### BRIEF DESCRIPTION OF THE DRAWINGS

Various aspects of systems and methods will now be presented in the detailed description by way of example, and not by way of limitation, with reference to the accompanying drawings, wherein:
Figure 1 is a block diagram of a system for assessing an effect of a medical treatment on an organ based on regional change measurements of the organ and regional treatment information associated with the medical treatment.
Figure 2 is a representation of regional treatment information associated with a medical treatment in the form of radiation therapy on a lung.
Figures 3A, 3B, and 3C are schematic illustrations of motion reconstruction for a region of an organ.
Figure 4 is a schematic diagram of a computed tomographic X-ray velocimetry (CTXV) system that captures medical images for use by the system, and may include one or more components of the system of Figure 1.
Figure 5A is a visual representation of a treatment effect determined by the system of Figure 1 in the form of a plot of regional change measurements, e.g., a comparison of specific ventilation before treatment and specific ventilation after treatment, against regional treatment information, e.g., radiation dose.
Figure 5B is a depiction of a two-dimensional (2D) slice of first measurements, e.g., the before-treatment specific ventilation measurements of Figure 5A, overlaid onto an image of a computed tomographic (CT) slice for visualization.
Figure 5C is a depiction of a 2D slice of second measurements, e.g., the aftertreatment specific ventilation measurements of Figure 5A, overlaid onto an image of a CT slice for visualization.
Figure 6A is a visual representation of another treatment effect determined by the system of Figure 1 in the form of a plot of regional change measurements, e.g., a comparison of specific ventilation before treatment and specific ventilation after treatment, against regional treatment information, e.g., radiation dose.
Figure 6B is a depiction of a slice of first measurements, e.g., the beforetreatment specific ventilation measurements of Figure 6A, overlaid onto an image of a CT slice for visualization.
Figure 6C is a depiction of a slice of second measurements, e.g., the aftertreatment specific ventilation measurements of Figure 6A, overlaid onto an image of a CT slice for visualization.
Figure 7 is a flow chart of a method of determining an effect of a medical treatment on an organ.
Figure 8 is a block diagram of an apparatus configured to implement the method of Figure 7.
Figure 9A is an example of regional treatment information in the form of a 2D slice of a three-dimensional dose map showing a spatial distribution of radiation dose administered during a medical treatment.
Figure 9B is a visual representation of the treatment effect of the radiation dose of Figure 9A four months after treatment, in the form a boxplot of regional change measurements, e.g., a comparison of specific ventilation before treatment and specific ventilation after treatment, against regional treatment information, e.g., radiation dose.
Figure lOA is an example of regional treatment information in the form of a 2D slice of a three-dimensional dose map showing a spatial distribution of radiation dose administered during a medical treatment.
Figure lOB is a visual representation of the treatment effect of the radiation dose of Figure lOA four months after treatment, in the form a boxplot of regional change measurements, e.g., a comparison of specific ventilation before treatment and specific ventilation after treatment, against regional treatment information, e.g., radiation dose.
Figure IOC is a visual representation of the treatment effect of the radiation dose of Figure lOA twelve months after treatment, in the form a boxplot of regional change measurements, e.g., a comparison of specific ventilation before treatment and specific ventilation after treatment, against regional treatment information, e.g., radiation dose.

### DETAILED DESCRIPTION

The methods, systems, and apparatus disclosed herein assess the effect of medical treatments on the lungs and other organs at a granular level. To this end, the methods and systems provide the capacity to measure organ function on a regional basis, and to compare or correlate regional organ function with regional treatment information, on a region by region basis. This enables a much richer and more complete understanding of an extremely complex treatment circumstances. The methods, systems, and apparatus enable the assessment of organs that are being treated, as well as other organs in the vicinity of the treated organ.

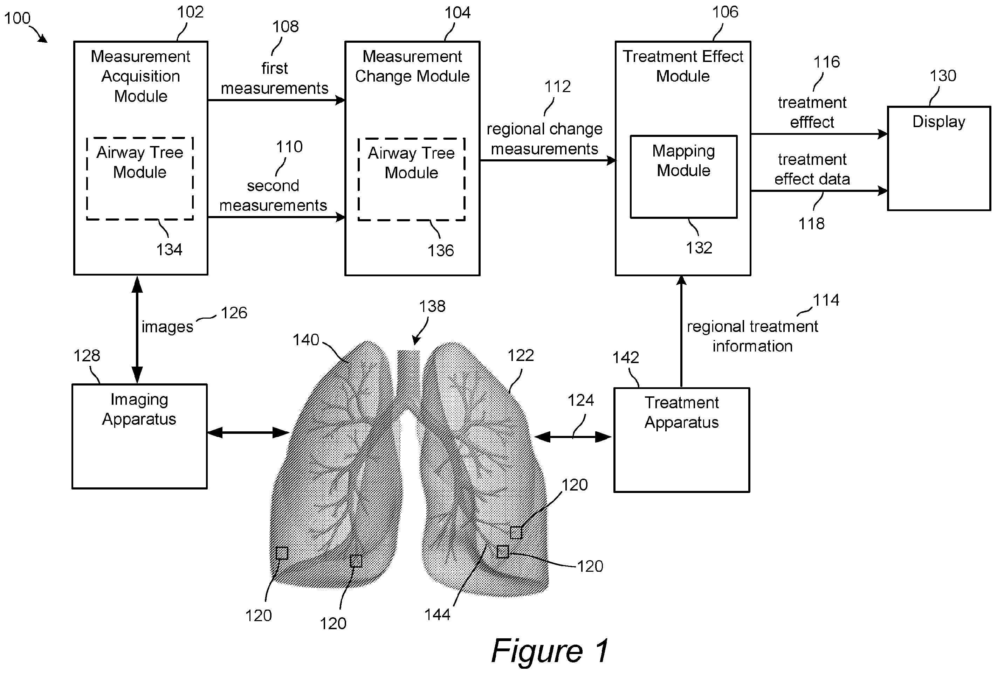

Figure 1 is a block diagram of a system 100 for assessing an effect 116 of a medical treatment 124 on an organ 122 of a patient based on changes in regional measurements 112 of the organ and regional treatment information 114 of the medical treatment. While a patient as used herein is a human, the system 100 may be used on animal subjects, or modeled living organs for use in an ex vivo or in vitro experiment. The organ 122 being assessed may be any anatomical structure that has motion associated with it, or blood or fluid flowing through it. For example, the organ 122 may be a lung, a heart, a gastrointestinal tract, a lymphatic system, a vascular system, a respiratory system. Furthermore, the organ 122 being assessed is not necessarily the entire organ. The system 100 may focus its assessment to structures or components of organs, such as the airways of the respiratory system, or arteries of the heart or vessels of the vascular system.

The effect 116, referred to herein as a "treatment effect," may correspond to an assessment of the overall function or a regional function of an organ 122 that has been subjected to a treatment 124, or an assessment of the function of an organ that is adjacent to or near an area of the body that has be subjected to treatment. The treatment effect 116 may correspond to a determination that a medical treatment 124 has either positively altered or affected, negatively altered, or affected, or had no effect on organ function.

In general terms, the treatment effect 116 is derived based on an association between changes in regional measurements 112 of an organ 122 due to the treatment 124, and corresponding regional treatment information 114 of the treatment. "Regional measurements" as used herein refers to measurements 108, 110 that are obtained for each of a number of individual regions 120 of an organ 122, as opposed to a single global measurement for the entirety of the organ. "Regional treatment information" as used herein refers to treatment information 114 for each of a number of individual regions 120 of the organ. "Region" as used herein corresponds to a part or portion of the organ less than its entirety, and generally a significantly small portion. A region 120 may be characterized in terms of system technology. For example, a region 120 may correspond to a physical part of an organ 122 equivalent in size to a two-dimensional (2D) display window (e.g. 16 x 16 pixels, or even a single pixel), or to a threedimensional (3D) display window (e.g. 8x8x8 voxels, or even a single voxel), or to a vector node.

Regarding "regional measurements," these measurements 108, 110 may be any type of measurement that results from the movement of fluid, e.g., air, blood, etc., through an organ. For example, in the case of the lung, the regional measurements 108, 110 may be ventilation measurements derived from volume or expansion measurements of 3D regions or voxels of tissue associated with an airway tree of the lung, which volume and expansion measurements are derived from motion measurements of tissue of the lung. In other words, motion measurements of regions of tissue are obtained first, and from these tissue motion measurements, relevant physiological measurements related to air flow, e.g., ventilation, may be derived. Ventilation measurement is intended to include both lung volume and the change in lung volume (e.g. specific ventilation, which is "change in volume" divided by "initial volume"), and can be measured at any point in a respiration cycle, including one or more points in an inspiration phase or portion of a respiration cycle and/or one or more points in an expiration phase or portion of a respiration cycle. For example, ventilation measurements may be made between start inspiration and end inspiration (i.e. peak inspiration), so that the measurements cover a full breath in. Ventilation measurements may be made during natural tidal breathing or at times corresponding to a desired period of the respiration cycle.

Regional measurements 108, 110 for the lung may also be perfusion or blood flow measurements, or a combination of ventilation and perfusion measurements (e.g. the ratio of ventilation and perfusion). Perfusion measurements in the lung may be derived from 3D images of the vascular structure of the lung or expansion measurements of 3D regions or voxels associated with the vascular structure of the lung in combination with further calculations and/or modelling.

In the case of other organs, such as the heart, the regional measurements 108, 110 may be blood flow measurements. For example, blood flow measurements may be derived from volume or expansion measurements of 3D regions or voxels associated with the various chambers of the heart or other vascular structures of the heart.

As described further below, the regional measurements 108, 110 may be acquired from a time series or sequence of medical images 126. In one embodiment, a sufficient number of medical images 126 of a patient are obtained and processed using a technique that measures the motion of the organ, such as a cross-correlation technique, to determine the regional measurements 108, 110. In other embodiments, fewer medical images may be obtained and the regional measurements may be determined through calculation or estimation or modelling (e.g. the field of computational fluid dynamics (CFD) provides for methods of calculating the flow of air through airways or blood through vasculature).

Regarding "medical treatments," these treatments 124 may involve one or more treatment types, modalities, or therapies, either non-invasive or invasive in nature. For example, the treatment 124 may be non-invasive radiation therapy, proton therapy, or drug therapy (including targeted drug therapies such as theragnostics), each delivered in accordance with a treatment regimen comprised of individual doses of treatment that are periodically, e.g., daily, weekly, monthly, etc., delivered to an organ over a period of time. Alternatively, the treatment 124 may be invasive and involve organ modifications or enhancements in the form of surgical dissection, tissue ablation, stent placement, valve placement, and glue application.

Some treatments 124 may be characterized as non-uniform in their delivery to the body in that the treatment targeted to a specific area of the body also effects surrounding areas of the body. For example, in the case of radiation therapy for cancer, a treatment plan may prescribe the delivery of a dose of radiation to a tumor at a target location in the body. Radiation delivery, however, is not perfectly delivered to the target. Accordingly, radiation exposure during treatment 124 is not limited to the target location. Areas surrounding or otherwise in the vicinity of the target are also exposed to radiation, although typically at a lower dose. As such, techniques such as radiation therapy tend to have a regional effect on organ function, meaning the therapy may have a positive effect on some regions of the organ 122, while having a negative effect on other regions of the organ. For example, cancerous tissue may be shrunk or killed in one or more regions of a lung, potentially increasing lung function in those regions. Conversely, non-cancerous tissue on other regions of the lung may be negatively affected by radiation dose. These effects, both positive and negative, are likely correlated to the dose of radiation delivered to the effected regions. Radiation therapy may also affect surrounding organs. For example, during radiation therapy treatment for breast cancer the heart may be inadvertently exposed to radiation. As such, the system 100 may be used to acquire regional blood flow measurements of the heart before and after the treatment to obtain a regional change measurement for each of a plurality of regions of the heart.

As another example of a non-uniform treatment, a stent implanted in a lung to open a narrow or blocked bronchi of the lung positively affects the regions of the lung at the implant site by increasing lung function in those regions. The stent, however, may negatively affect surrounding regions of the lung, for example, by causing a wall of an adjacent bronchi to partially collapse, and thereby decreasing lung function in the regions of the adjacent bronchi. Likewise, a stent implanted in a coronary artery of the heart positively affects the regions of the artery at the implant site by increasing blood flow in those regions and improving cardiac function. The stent, however, may negatively affect surrounding regions of the heart, for example, by causing a wall of an adjacent artery to partially collapse, and thereby decreasing blood flow in the regions of the adjacent artery. Other examples include the delivery of treatment through carriers that can either be directly placed in the region of interest. Examples include the placement of radioactive beads within blood vessels that feed a liver cancer, or theranostics (whereby treatment and diagnostic imaging substances are combined with a substance which is attracted to or binds with certain targets within the body).

Regarding "regional treatment information," this information 114 may be created by or result from a targeted treatment of a region of an organ 122. For example, a medical treatment 124 in the form of radiation therapy attempts to deliver a specified dose of radiation to the diseased area in accordance with a radiation therapy treatment plan. The radiation therapy treatment plan provides a detailed knowledge of the dose planned to be delivered to each region 120 of the lung 122 during a treatment 124. The radiation treatment plan is determined in advance of the treatment, using known techniques. Alternatively, radiation (or other treatments) delivered through a theranostics approach can be estimated or measured using for example PET imaging, or soon after such treatment, using for example molecular nuclear imaging. In either case, the regional treatment information 114 associated with a treatment 124 may be represented by a dose map.

Regional treatment information 114 may also be created through a separate process remote from the diseased area. For example, lung valves or lung stents are typically implanted in the airway tree at a location upstream from the diseased or unhealthy area. Thus, in these types of interventions, treatment in one region of the organ affects other regions of the organ. Regional treatment information 114 showing the regions of the organ where the device is implanted may be known with certainty by the surgeon or medical practitioner, or acquired from medical imaging of the device after implant. Devices that release treatment substances (e.g. drugs) or radiation, may also require additional steps of calculating the resultant delivery (e.g. radiation typically reduces with the square of the distance from the device delivering the radiation).

In accordance with embodiments disclosed herein, regional treatment information 114 on a particular treatment may be available in the form of a treatment map that associates a treatment parameter with each of a number of regions 120 of an organ, e.g., a lung 122. For example, in the case of radiation therapy for a lung 122, the treatment map may be in the form of a data set that lists each region 120 of the lung by, for example, 3D coordinates, and a corresponding radiation dose either delivered to or expected to be delivered to that region. Generally, the doses listed in a treatment map will be higher for those regions of the lung 122 that are at or immediately around the target tumor, and will progressively reduce in value for other regions as a function of distance from the target tumor.

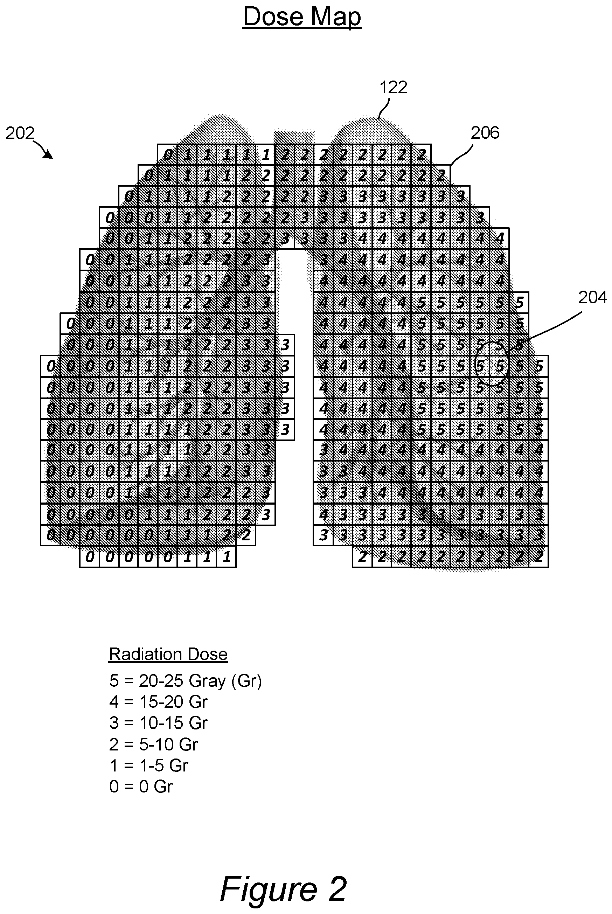

Figure 2 provides a visual representation of a dose map 202 for a radiation treatment delivered to a lung 122 having a tumor 204. A grid of treatment regions 206 overlays a visual representation of the lung 122. Each treatment region 206 corresponds to a region 120 of the lung 122. The number 1-5 in each treatment region 206 represents the dose of radiation delivered to that region 120 of the lung 122, with the greater number corresponding to a higher dose of radiation. While the visual representation of the dose map 202 is two-dimensional, the dose map 202 is threedimensional in nature, with the treatment regions 206 corresponding to cubes or voxels that extend depth-wise into the lung 122. It is noted that doses delivered to treatment regions 206 near the tumor 204 are greatest, with the doses progressively reduce as a function of distance from the tumor.

Returning to Figure 1, the continuing description of the system 100 is in terms of an organ corresponding to a lung and treatment in the form of radiation for purposes of treating lung cancer. Radiation treatment of a lung may involve a delivery of a prescribed dose of radiation to a target location of a lung. As previously mentioned, a "dose" refers to a specified amount of treatment, e.g., radiation level/rate, delivered during one treatment session, and a "dosage" refers to a specified number of doses to be delivered at a specified frequency over a specified period of time. For example, in treating lung cancer, the prescribed dose may be delivered to a target tumor at daily treatment sessions that occur over a six week period.

The system 100 for assessing an effect of a medical treatment on an organ includes a measurement acquisition module 102, a measurement change module 104, and a treatment effect module 106. The system 100 may interface with an imaging apparatus 128 for purposes of acquiring images 126 of the organ, and a treatment apparatus 142, or other treatment information source, for purposes of acquiring regional treatment information 114. To these ends, the system 100 may be configured to acquire images 126 and treatment information 114 directly from an imaging apparatus 128 or treatment apparatus 142, or from another image source or treatment information source such as a cloud based server/database, or other computer network structure that stores images and treatment information. Alternatively, one or more of modules of the system 100 may comprise one or more of an imaging apparatus and a treatment apparatus. For example, the measurement acquisition module 102 may include an imaging apparatus.

The measurement acquisition module 102 is configured to acquire a first measurement 108 and a second measurement 110 for each of a plurality of regions 120 of a lung 122 based on images 126 of the lung acquired by the measurement acquisition module 102 from the imaging apparatus 128. The plurality of regions 120 could be as few as two regions, but would typically be more than 20 regions, more than 50 regions, more than 100 regions, more than 200 regions, more than 500 regions, or more than 1000 regions. In one embodiment, the images 126 are 2D images. In other embodiments, the images 126 may be 3D images.

The timing of acquisition of the first measurements 108 and the second measurements 110 relative to a treatment 124 may take on various scenarios. For example, the first measurements 108 may be acquired prior to a first delivery of a treatment, i.e., before the organ has ever been treated, or after the organ has been subjected to a treatment - but before the organ has been subjected to another treatment. The first measurements 108 may be acquired on the same day of a treatment 124, or even during a delivery of a treatment 124. The second measurements 110 are acquired after acquisition of the first measurements 108 and after or possibly during a delivery of a treatment 124 to the lung 122 by the treatment apparatus 142. For example, the second measurements 110 may be acquired immediately after delivery of a treatment 124, or a time after the treatment delivery sufficient to allow the effect of the treatment to manifest in the organ. Alternatively, the second measurements 110 may be acquired during delivery of a treatment 124. Acquisition during a treatment 124 preferably occurs for treatments whose effects on organs are expected to be immediate.

What is important is that the first measurements 108 are acquired before the effects of the treatment 124 manifest (in order to create a baseline of organ function) and the second measurements 110 are acquired some time after the first measurements 108 (e.g. after the effects of the treatment are expected to manifest). It will be understood that these timelines are different for different procedures. For example, in the case of radiation therapy on the lung (and monitoring for radiation induced pneumonitis, a side effect of unwanted radiation exposure in the lung), the first measurements 108 might be acquired on the same day of treatment, before the pneumonitis sets in, while the second measurements 110 would be acquired substantially after treatment (e.g. 1 or more months later). In contrast, in the case of implanting a medical device during surgery, the first measurements 108 may be acquired some time before the operation (e.g. 1 week) in order to establish a baseline of organ function, and the second measurements 110 could be acquired immediately after implantation of the medical device (e.g. even while still in surgery), or alternatively some time after surgery (e.g. the next day).

The first measurements 108 and the second measurements 110 may be one of: regional lung displacement measurements, regional lung velocity measurements, lung ventilation measurements, lung perfusion measurements, lung ventilation/perfusion (V/Q) ratio measurements, lung compliance measurements, or any measurements that may be derived from any of the foregoing measurements. For example, in the field of pulmonology, airway flow, pulmonary compliance, time constants, pulmonary resistance or air trapping measurements may be derived from lung ventilation measurements. An example of a lung ventilation measurement is a specific ventilation measurement, which as explained further below, corresponds to a measure of volume expansion of a region of the lung relative to the volume of that lung region. The first measurements 108 across the plurality of regions of the lung may be referred to as a first-measurement dataset, while the second measurements 110 across the plurality of regions of the lung may be referred to as a second-measurement dataset.

Continuing with Figure 1, in one embodiment the measurement acquisition module 102 acquires each of the first measurements 108 and the second measurements 110 by obtaining a time series or sequence of 2D images 126 of the lung 122, and processing the time series of 2D images 126 to obtain a motion measurement for each of the plurality of regions 120 of the lung. The measurement acquisition module 102 may obtain the time series of 2D images by receiving the images from an imaging apparatus 128, which may be an apparatus that captures the images, or an image source, e.g., a picture archiving and communication system (PACS), that stores the images for download. The imaging apparatus 128 may be a separate physical structure or it may be included in the measurement acquisition module 102, or any other module of the system 100. In any case, images 126 as used herein may correspond to image data or image datasets from which a visual image may be created. While these image datasets can be converted to visual images, it is understood that the processing of the images by the system 100 is usually with respect to the datasets.

The time series of 2D images 126 of the lung 122 may include a single time series of 2D images of the lung captured from one angle or perspective relative to the lung during all or a portion of a respiration cycle. A single time series of 2D images 126 of the lung at a particular angle may include a series or sequence of 2D images, where each respective image in the sequence is captured at a respective different time during (or phase of) inspiration or expiration or during an entire breath (both inspiration and expiration). Additional description of the foregoing acquisition of a time series or a sequence of 2D images 126 is included in U.S. Patent No. 10,674,987, titled "Method of Imaging Motion of an Organ."

The time series of 2D images 126 of the lung 122 may include a plurality of time series of 2D images of the lung, where each of the plurality of time series of 2D images is captured from a different angle or perspective relative to the lung, and during all or a portion of a respiration cycle. In this case, each of the plurality of time series of 2D images 126 of the lung 122 include a series of 2D images captured at a unique angle and at spaced apart times during inspiration or expiration. In one configuration, each of the plurality of time series of 2D images 126 of the lung 122 are captured from at least three different angles (in order to create a spread of angles). For example, the 2D images 126 of the lung 122 may be acquired from four angles or five angles, but in any case, preferably no more than ten different angles. Each of the plurality of time series of 2D images 126 of the lung 122 can be captured asynchronously within the same breath, simultaneously, or during different breaths, or any combination thereof.

The time series of 2D images 126 of the lung 122 may be obtained by the measurement acquisition module 102 from an imaging apparatus 128 that relies on X- rays to capture the images. For example, the imaging apparatus 128 may be fluoroscopy device, capable of capturing a time-series of 2D x-ray images. Alternatively, the 2D images 126 may be obtained from other suitable types of 2D medical imaging apparatuses, such as a projection MRI imaging apparatus, a mm-wave imaging apparatus, an infrared imaging apparatus, a four-dimensional CT imaging apparatus, or a positron emission tomography (PET) imaging apparatus. Additional description of the foregoing capturing of a time series or a sequence of 2D images 126 is included in U.S. Patent No. 10,674,987, titled "Method of Imaging Motion of an Organ", the entirety of which is incorporated herein by reference.

After acquisition of the 2D images 126, the measurement acquisition module 102 analyzes the images to calculate a first measurement 108 or a second measurement 110, e.g., ventilation, of the lungs. The motion of a region 120 of a lung 122 can be calculated by the measurement acquisition module 102 using any suitable technique, however in one embodiment it is measured using Computer Tomographic X-ray Velocimetry (CTXV) and a cross-correlation technique, as described in U.S. Patent No. 9,036,887 B2, titled "Particle Image Velocimetry Suitable for X-ray Projection Imaging", the entirety of which is incorporated herein by reference. CTXV uses X-ray images taken from multiple projection angles in order to measure regional threedimensional motion of the object, in this case the lungs. The motion tracking in CTXV is based on a well-known technique called particle image velocimetry (PIV), in which the displacement of a region is calculated by selecting a region in the first image of a time series and statistically correlating the selected region to the second image in the time series. The motion measurements can therefore be 2D or 3D measurements of displacement, velocity, expansion (or ventilation), or any other suitable motion measurement. The flow in the airways can also be calculated from the motion measurements.

Generally, using the cross-correlation technique, as described in U.S. Patent No. 9,036,887, a first measurement 108 or a second measurement 110 for a region 120 of the lung 122 is calculated by reconstructing motion measurements for each of the plurality of regions of the lung from the plurality of time series of 2D images 126 of the lung, and then deriving a volume or expansion measurement from one or more motion measurements associated with that region for each of the plurality of regions of the lung. In one embodiment, the reconstructing of motion measurements includes reconstructing 3D motion measurements without first reconstructing a 3D image.

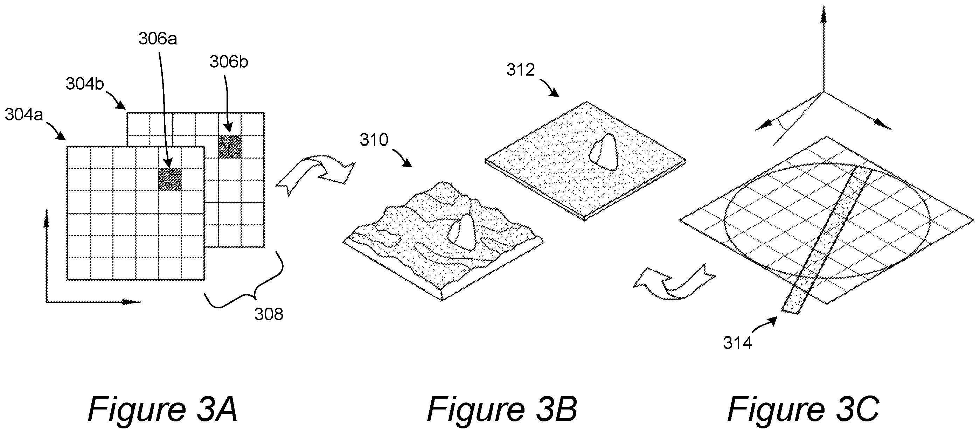

Further to the foregoing general description, and with reference to Figures 3A, 3B, and 3C, the measurement acquisition module 102 is configured to determine a 2D cross correlation for each of the plurality of time series of 2D images. To this end, the measurement acquisition module 102 splits a first image 304a in a time series 308 of 2D images into windows 306a, and then compares each window from the first image 304a to a corresponding window 306b of the second image 304b to determine where the window has moved to during the time between the first image and the second image, and how well the window 306a from the first image 304a correlates with the window 306b from the second image 304b. A 3D representation of the measured crosscorrelation 310 of windows 306a and 306b is shown in Figure 3B.

Based on the measured 2D cross-correlations, the measurement acquisition module 102 estimates what the 3D velocity flow field would have been for those measured 2D cross-correlations to have been produced. Next, the measurement acquisition module 102 determines modeled cross correlations 312 for the 3D estimate of the velocity flow field (a 2D representation 314 of an estimated cross-correlation of windows 306a and 306b is shown in Figure 3C.). In other words, the measurement acquisition module 102 calculates the cross correlations 312 that would occur for the estimated 3D flow. Note these estimated cross correlations 312 are not the same as the measured 2D cross correlations 310. Next, the measurement acquisition module 102 compares the measured 2D cross correlations 310 to the estimated cross correlations 312, and minimizes the error between the two using an iterative method, such as the Levenberg-Marquardt algorithm (which is a non-linear least-squares solver), to modify the estimated 3D velocity flow field (after which new estimated cross correlations are re-calculated).

When the error between the measured cross-correlations 310 and the estimated cross-correlations 312 has been fully minimized, the measurement acquisition module 102 has reconstructed a 3D motion field (i.e. the final estimated 3D velocity filed), without ever reconstructing a 3D image. This technique is typically referred to as Computed Tomography X-ray Velocimetry (CTXV), which is an extension of Particle Image Velocimetry (PIV). The measurement acquisition module 102 then calculates the (regional) expansion (also referred to as the ventilation, or specific ventilation) from the 3D motion field. This is done using a well known equation (du/dx -t dv/dy -t dw/dz).

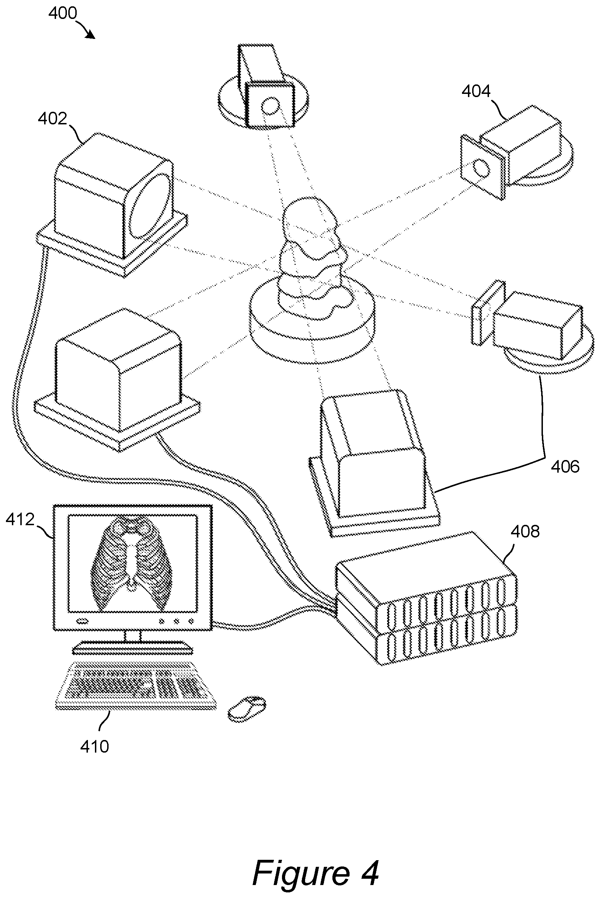

With reference to Figure 4, in one embodiment the measurement acquisition module 102 is included in a computed tomographic X-ray velocimetry (CTXV) system 400, along with an imaging apparatus 128. The CTXV system 400 includes imaging hardware, and image capture and analysis hardware and software. The imaging hardware includes a video speed or double shutter X-ray camera 402, a cone beam X-ray source 404, a source modulation system 406, basic source alignment and high-resolution camera alignment hardware 408, image capture and analysis hardware 410, and a user interface 412. The image capture and analysis hardware and software would typically consist of the following key components: high speed image capture hardware; high speed image processing hardware, image processing software and user interface for alignment, imaging, and analysis. Details of the CTXV system 400 are described in U.S. Patent No. 9,036,887 B2, titled "Particle Image Velocimetry Suitable for X-ray Projection Imaging." .

Returning to Figure 1, the measurement change module 104 is configured to obtain a regional change measurement 112 for each of the plurality of regions 120 of the lung 122 based on the first measurement 108 and the second measurement 110 of the region. To this end, the measurement change module 104 may be configured to compare, on a region 120 by region basis, the first measurement 108 of the region to the second measurement 110 of the region. The comparison may be based on any one of various forms of mathematical or statistical analysis. For example, the comparison may be a difference between the first measurement 108 of the region to the second measurement 110 of the region obtained by subtracting the first measurement from the second measurement. Or the comparison may be a percentage change between the first measurement 108 of the region to the second measurement 110 of the region. Or the comparison may be the average of the first measurement 108 of the region and the second measurement 110 of the region. Also, if a number of additional second measurements are obtained overtime, e.g., one after each of a series of radiation treatments (as could be done for monitoring the effect of a treatment over time), the comparison may involve a mathematical or statistical analysis of all measurements overtime. The comparison may be, for example, a fitted curve that relates the series of measurements and represents a trend in the effect on that region. The regional change measurements 112 across the plurality of regions of the lung may be referred to as a change-measurement dataset.

These regional change measurements 112 provide information about the change in ventilation for each region 120, thereby allowing for changes in ventilation to be more readily identified. For example, a negative regional change measurement 112, e.g., a negative change in specific ventilation, indicates that the patient's ventilation (surrogate measurement for lung health, or lung capacity) has decreased or declined in the region corresponding to the regional change measurements 112. Conversely, a positive regional change measurement 112 e.g., a positive change in specific ventilation, indicates that the patient's ventilation has increased or improved in that region.

Continuing with Figure 1, the treatment effect module 106 is configured to obtain a treatment effect 116 based the plurality of regional change measurements 112 and the regional treatment information 114 of the treatment 124 delivered to the lung 122. As previously mentioned, regional treatment information 114 of a treatment 124 may be obtained by the treatment effect module 106 directly from a treatment apparatus 142, or from other treatment information sources, such as a cloud based server/database, or other computer network structure that stores the relevant treatment information.

The treatment effect 116 may indicate whether the treatment 124 has affected overall lung function, or on a more granular level, whether the treatment has affected some regions 120 of the lung 122 more than other regions of the lung. The treatment effect 116 may also indicate whether a change in lung function is the result of treatment or not. For example, a treatment effect 116 may indicate a) no change in lung function, b) a change in lung function linked to treatment, or c) a change in lung function not linked to treatment.

More specifically, in terms of lung ventilation, a treatment effect 116 may indicate that there has been no ventilation change (e.g. the function of the lung 122 has not changed). This treatment effect 116 may arise, for example, when the regional change measurements 112 show no ventilation change. In this case a patient receiving treatment for lung cancer, and a doctor or the system 100 monitoring for a reduction in lung function due to unwanted additional radiation exposer in the lungs can be satisfied with the patient's lung health (as there has been no change in ventilation).

A treatment effect may indicate that there has been ventilation change and it is linked to treatment (e.g. ventilation change, either a reduction or an increase, has occurred in an area of the lung 122 that corresponds to the regional treatment information 114). This treatment effect 116 may arise, for example, when a patient has received a radiation dose to a particular region of the lung 122, and the regional change measurements 112 show a reduction in the ventilation. In this case, a physician, or the system 100 itself may deduce that the altered lung heath of the patient is due to the radiation therapy delivered to the lung 122.

A treatment effect may indicate that there has been ventilation change but that is not linked to treatment (e.g. ventilation change, either a reduction or an increase, has occurred in an area of the lung 122 that does not correspond to the regional treatment information 114). This treatment effect 116 may arise, for example, when a patient has a general reduction in ventilation throughout the lungs indicated by the regional change measurements 112, or a localized reduction in an area of the lung not associated with the regions of the lung to which the radiation was delivered. In this case, the physician, or the system 100 itself may deduce that the radiation therapy is not the cause of the reduction in lung function, and may look to other potential causes, such as pneumonia.

The ability to quickly identify the root cause of a change in lung function is critical for physicians, as different root causes require different treatments (and any delay in treatment can lead to progression of the disease). In other words, the system 100 is of particular use for determining whether a treatment has altered regional lung function. This alteration in lung health may be used to assess the effectiveness, or efficacy, of a treatment (e.g. an increase in lung function at the site of a treatment could indicate that the treatment is being successful), or to assess whether there have been any negative impacts of the treatment (e.g. a decrease in lung function at the site of a treatment could indicate that the treatment is has caused negative side effects).

Returning to Figure 1, the treatment effect module 106 may include a mapping module 132 that is configured to map (e.g. register) each of the plurality of regional change measurements 112 with a corresponding regional treatment information 114 of the treatment delivered to the lung 122. In other words, for each region 120 of the lung 122, the regional treatment information 114, e.g., dose map information from a radiation therapy treatment as shown in Figure 2, for a region is mapped to the regional change measurement 112 for that region.

The mapping may include registration processes such as transformation, deformation, rotation, interpolation etc. of the dataset of regional change measurement 112 and/or the dataset of regional treatment information 114 to ensure proper overlap of the regions. For example, in some instances the regional treatment information 114 provided in a dose map and the regional change measurements 112 may not be in the same physical position, e.g. the x, y, z position of the top of the left lung could be 0, 0, 0 in the dose map, but 12, 15, 28 in the regional change measurements. To address this, the mapping module 132 is configured to translate one or both of the regional change measurements 112 and the regional treatment information 114, e.g., dose map, until the respective physical positions overlap/correspond correctly.

The mapping module 132 may also be configured to deform or scale one or both of the regional change measurements 112 and the regional treatment information 114 in cases where the voxel sizes of the two datasets were different. The mapping module 132 may also be configured to rotate one or both of the regional change measurements 112 and the regional treatment information 114 to the same angle if they were acquired from different angles. The mapping module 132 may also be configured interpolate one or both of the regional change measurements 112 and the regional treatment information 114 if they were acquired at different resolutions.

In any case, this mapping by the mapping module 132 provides for each region 120 of the lung 122, a measurable comparison of the function of that region before treatment 124 relative to the function of that region after treatment, and further as a function of the regional treatment information 114 for that region. The mapping results in treatment effect data 118.

The treatment effect module 106 may then derive the treatment effect 116 from the treatment effect data 118 and output the treatment effect for observation by the system user, e.g., physician. The treatment effect module 106 may be further configured to provide the treatment effect data 118 to a display 130 to enable user interpretation of the data and user determine treatment effects.

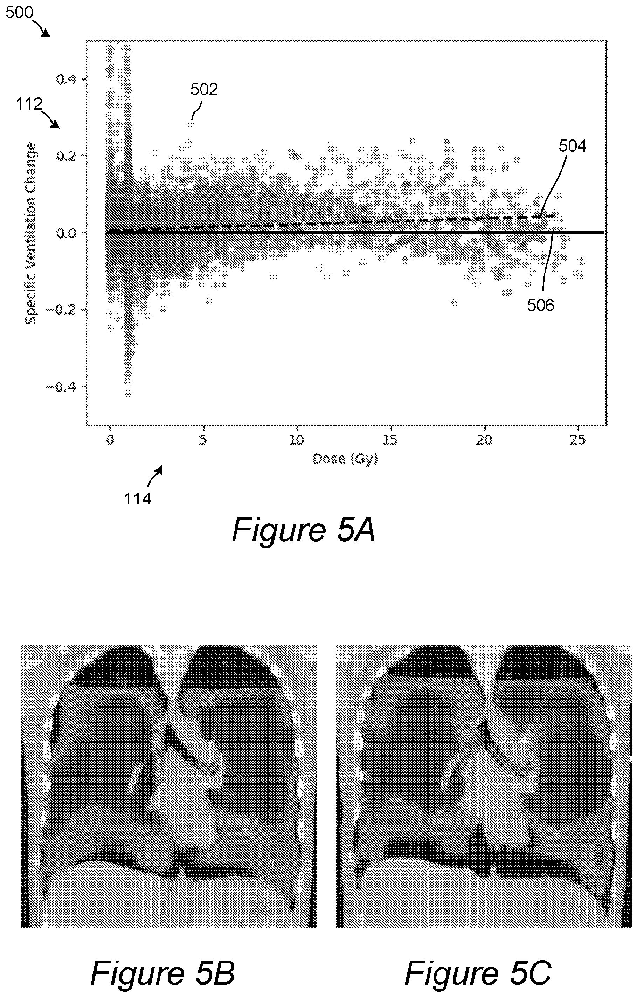

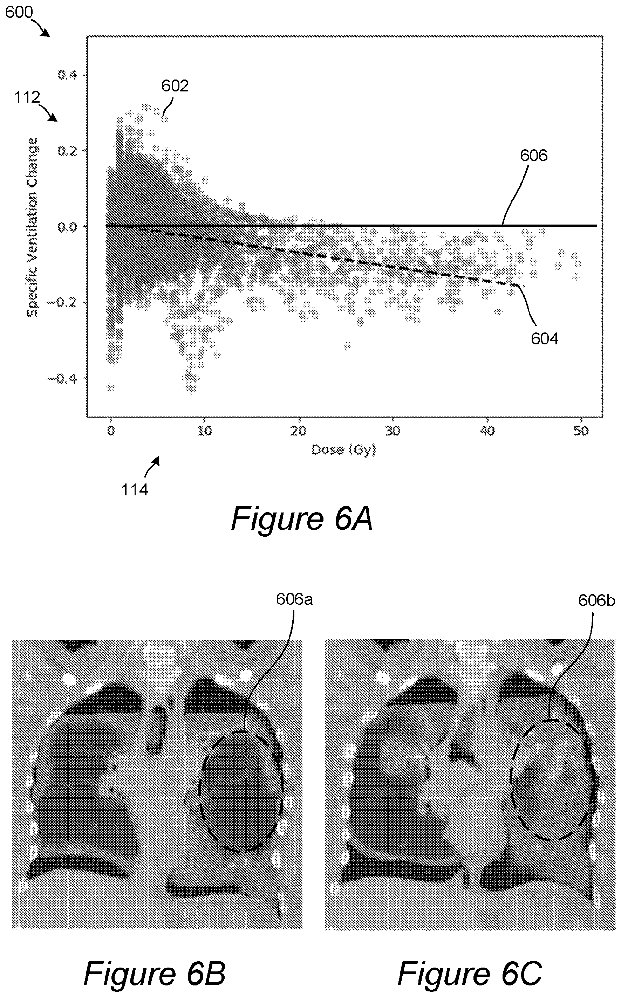

With reference to Figures 5A and 6A, treatment effect data 118 may be represented by a plot 500, 600 of data points 502, 602, where each point represents a region 120 of the lung. The location of a regional data point 502, 602 is based on the regional change measurement 112 of the region 120 and the regional treatment information 114 of the region. In Figures 5A and 6A, the regional change measurements 112 are specific ventilation changes corresponding to a difference between a first specific ventilation measurement for a region 120 and a second specific ventilation measurement for the same region. The regional treatment information 114 are x-ray doses delivered to each region 120 of the lung 122, as provided for example by a does map like shown in Figure 2. It is noted that the plotted regional change measurement dataset and the regional treatment information dataset are both 3D datasets, resulting in many separate data points 502, 602 to plot. The collection of data points 502, 602 may be referred to as a comparison dataset.

With continued reference to Figures 5A and 6A, in one configuration, the treatment effect module 106 is configured to fit the data points 502, 602 with a line 504, 604 and to analyze the line to arrive at a treatment effect 116. To this end, the treatment effect module 106 may determine the slope of the fitted line and evaluate it against a criterion to determine if a trend (correlation) exists in the data points 502, 602.

For example, the treatment effect module 106 may be configured to detect a fitted line having a slope or gradient in a specified direction, e.g., negative or positive, and to generate a treatment effect 116 accordingly. In the case of a positive slope, the treatment effect module 106 may be programmed to output a treatment effect 116 in the form of a message indicating that "treatment has not negatively affected lung function." A case such as this is described further below with reference to Figure 5A. In the case of a negative slope, the treatment effect module 106 may be programmed to output a message for display indicating that "treatment has negatively affected lung function." A case such as this is described further below with reference to Figure 6A.

In Figure 5A, the treatment effect module 106 has fitted a linear line 504 to the data points 502, but any other suitable line could have been used to fit the data. As can be seen in Figure 5A, the fitted line 504 has a very slight positive gradient, indicating that the radiation dose from the treatment 124 has not had a negative impact on the ventilation of the patient's lungs. In other words, while some regions 120 of the lung 122 exhibit significant changes in specific ventilation, e.g., > +0.1, the majority of the regions exhibit little to no change, e.g., < ± 0.1. Most significantly, and leading to a positive gradient of line 504, a majority of regions 120 of the lung 122 that were exposed to the higher dose of radiation, e.g., between 10-25 Gy, experienced an increase in specific ventilation - as represented by data points 502 above the zero line 506.

Referring to Figure 6A, the fitted line 604 has a definite negative gradient, indicating that the radiation dose from the treatment 124 has had a negative impact on the ventilation of the patient's lungs. Most significantly, and leading to a negative gradient of line 604, a majority of regions 120 of the lung 122 that were exposed to radiation dose between 10-50 Gy, experienced a decrease in specific ventilation - as represented by data points 602 below zero line 606.

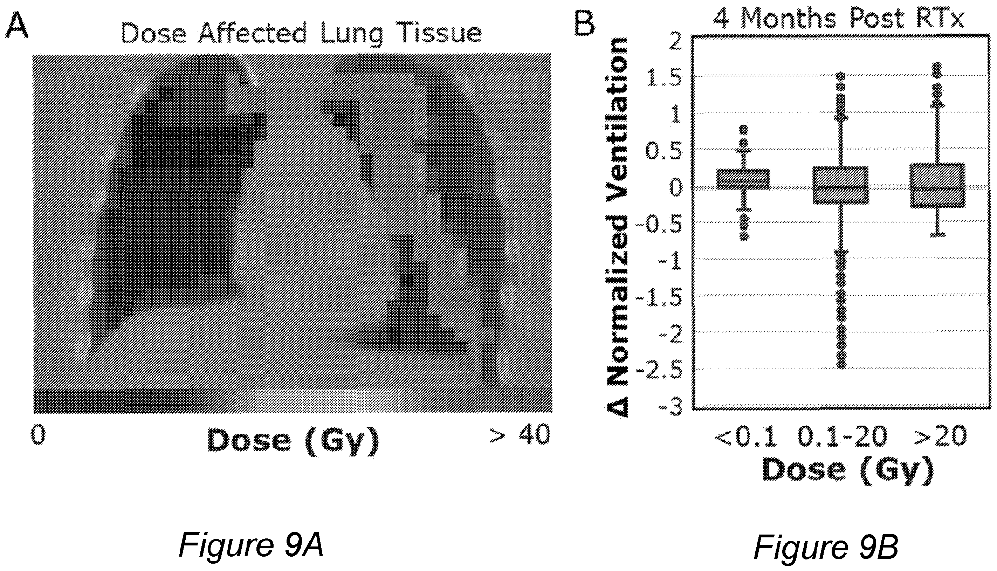

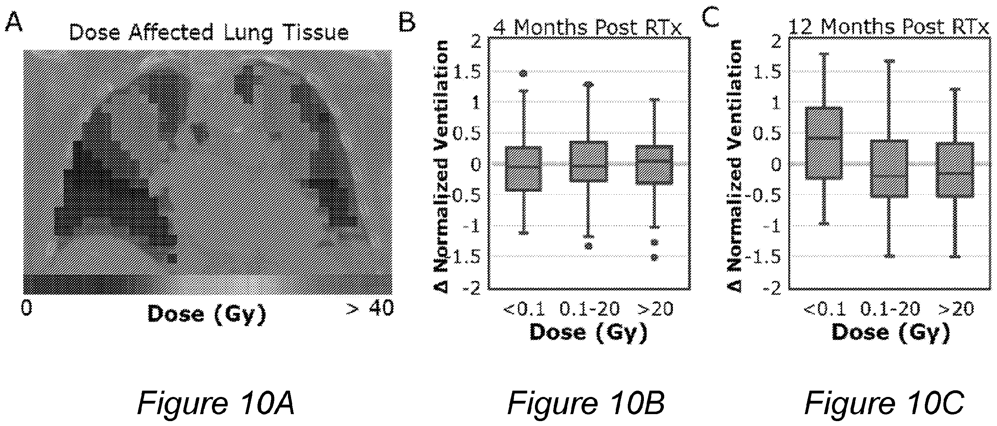

While the examples in Figure 5A and 6A describe the analysis of a fitted line 504, 604 to generate a treatment effect 116, it is understood that other types of data analyses of the regional change measurements 112 and regional treatment information 114 are possible. For example, as described later below with reference to Figures 9B, lOB, and IOC, an analysis of boxplots of regional change measurements 112 verses regional treatment information 114 may be used to generate a treatment effect 116. Alternatively, instead of representing the treatment effect data 118 as a 2D plot, the treatment effect module 106 may combine the regional change measurements 112 and the regional treatment information 114 into a single dataset by, for example, multiplying the change measurement 112 of each region with the treatment information 114 of the corresponding region. This would result in regions that have both a high change measurement value and a high treatment information value being more pronounced (i.e. if a region had a high radiation dose that resulted in a significant decrease in ventilation, the combined value of that region would be high). Such data could be displayed as a 2D slice of the three-dimensional data (for example, in the style shown in Figure 5B).

As previously mentioned, the treatment effect module 106 may be configured to provide treatment effect data 118 to a display 130 to enable user interpretation of the data and the user to determine the treatment effects. The treatment effect data 118 may also be output in the form of a physical report. For example, the treatment effect module 106 may output the treatment effect data 118 to enable a display of the plots of Figures 5A and 6A on the display 130. In addition, the treatment effect module 106 may be configured to forward image data corresponding to the first measurements 108 and the second measurements 110 to the display 130 to facilitate a manual comparison by displaying before and after images that enable a side-by-side visual comparison in which the first measurements may be compared to the second measurements.

For example, in displayed lung images corresponding to Figures 5B and 5C it can be seen that there is no substantial difference between the first measurements 108 shown in Figure 5B and the second measurements 110 shown in Figure 5C. In other words, there is no substantial change in the regional change measurements 112. For displayed lung images corresponding to Figures 6B and 6C it can be seen that there is a substantial difference between the first measurements 108 shown in Figure 6B and the second measurements 110 shown in Figure 6C, in particular when comparing region 606a of the first measurements 108 to the same region 606b in the second measurements 110.

From the foregoing it is noted that treatment effects 116 automatically determined by the system 100 based on regional change measurements 112 and regional treatment information 114, and the provision of accompanying treatment effect data 118, provide information on the efficacy of a treatment. This information enables judgments to be made as to how a treatment has affected the lungs. These judgements, may be made by humans, e.g., doctor/physician, researcher, based on visual observations of treatment effect data 118, such as plots like those shown in Figures 5A and 6A. Alternatively, the judgements may be made automatically by the system 100 through its processing of regional change measurements 112 and regional treatment information 114.

As previously mentioned, treatments may often have an effect at a distance. For example, treatment of lung cancer may shrink a tumor occluding an airway. Alternatively, the placement of a lung valve may alter the flow in an airway. Similarly, the placement of a lung stent may alter an airway. These treatments will have their largest effect on the tissue distal to that airway/blood vessel. However, by associating the change in function with the airway tree/vascular tree etc. the changes can be compared at the airway/vasculature level, rather than at the tissue level, but still in a similar fashion to direct/local effects on tissue. To this end, additional embodiments of the system 100 focus on the processing and analysis of measurements associated with the airway trees of the lungs.

With reference to Figure 1, in another embodiment of the system 100, the measurement acquisition module 102 includes an airway tree module 134. The airway tree module 134 is configured to further process the first measurements 108 and the second measurements 110 to associate them with a fluid flow structure, e.g., an airway tree 138, of the lung 122. In other words, the airway tree module 134 takes a first type, e.g., lung tissue motion measurements, of the first measurements 108 and the second measurements 110 and converts them into a second type, e.g., airway flow measurements, by associating each region 120 of lung tissue with a specific airway or branch 144 of the airway tree 138.

It will be understood that the airway tree module 134 could be any other type of module for associating, modifying or converting the first measurements 108 and the second measurements 110 to associate them with a fluid flow structure. For example, when measuring blood flow of the lung, the airway tree module 134 could instead be a vascular tree module for extracting vasculature flow measurements. Within the context of other organs, such as the heart, the airway tree module 134 could instead be a vascular structure module for associating, modifying or converting the first measurements 108 and the second measurements 110 to associate them with a fluid flow structure, e.g., a vascular structure, of the heart such as the cardiac chambers, coronary vessels, etc.

Returning to the airway tree module 134, the first measurements 108 are associated with an airway tree 138 to create a set of first measurements 108 referred to as first airway flow measurements. Similarly, the second measurements 110 are associated with the airway tree 138 to create a set of second measurements referred to as second airway flow measurements. The first airway flow measurements and second airway flow measurements are forwarded from the measurement acquisition module 102 to the measurement change module 104, and are processed by the measurement change module 104 in the same manner described above, to create regional change measurements 112 referred to as regional airway flow change measurements. For example, the second airway flow measurements in one branch 144 of the airway tree 138 can be subtracted from the first airway flow measurements in the same branch, thereby creating airway flow change measurements for that branch. This process can be repeated for all branches 144. The regional airway flow change measurements are forwarded from the measurement change module 104 to the treatment effect module 106, and are processed by the treatment effect module 106 in the same manner described above to determine a treatment effect 116.

The airway tree 138 can be created by segmenting and skeletonizing the airway from a CT image, or from any other suitable method. The first measurements 108 and the second measurements 110 can be associated with the airway tree 138 using any suitable method. For example, the skeletonized airway tree may be inspected/interrogated to locate the endpoints 140 of each branch 144 of each airway, and then each first measurement 108 and each second measurement 110 can be allocated to its nearest endpoint 140. Summation of the measurements back up the tree, e.g., from the endpoint 140 to the beginning of the airway, i.e. the mouth, provide the airway flow throughout the airways. Such segmenting and skeletonizing techniques are described in U.S. Patent Application Publication No. US 2020/0069197, titled "Method of Scanning and Assessing Lung and Vascular Health", the entirety of which is incorporated herein by reference.

With reference to Figure 1, in another embodiment of the system 100, the measurement change module 104 includes an airway tree module 136. The airway tree module 136 is configured to further process the regional change measurements 112 to associate them with an airway tree 138 of the lung 122. In other words, the airway tree module 136 takes a first type, e.g., regional lung tissue motion measurements, of regional change measurements 112 and converts them into a second type, e.g., regional airway flow change measurements, by associating each region 120 of the regional lung tissue change measurements 112 with a specific airway or branch 144 of the airway tree 138.

Again, it will be understood that the airway tree module 136 could be any other type of module for associating, modifying or converting the regional change measurements 112. For example, when measuring blood flow, the airway tree module 136 could instead be a vascular tree module for extracting regional vasculature flow change measurements.

Returning to the airway tree module 136, prior to being output to the treatment effect module 106, the regional change measurements 112 are associated with an airway tree 138 to create a set of regional change measurements referred to as regional airway flow change measurements. The regional change measurements 112 can be associated with the airway tree 138 in the same manner described above with respect to the first measurements 108 and the second measurements 110. The regional airway flow change measurements are then processed by the treatment effect module 106 in the same manner described above to determine a treatment effect 116.

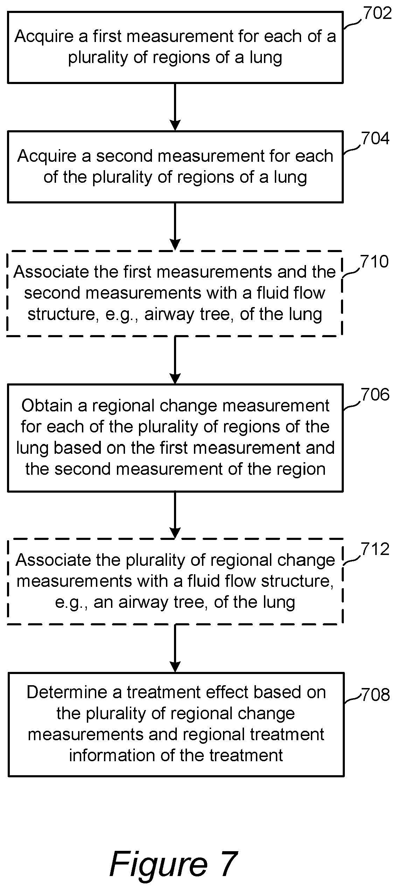

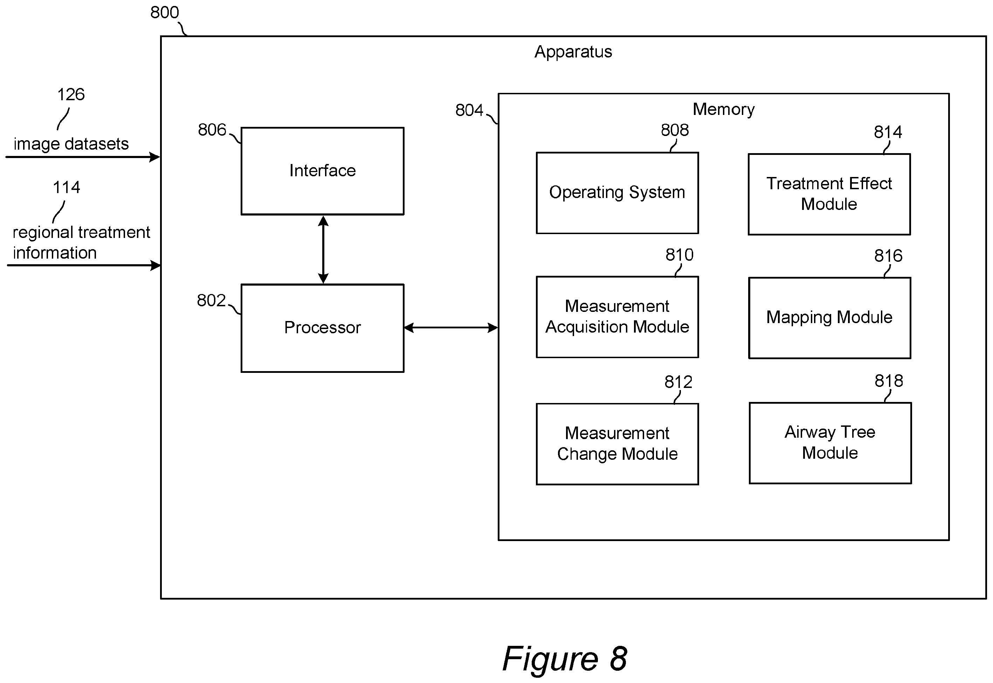

Figure 7 is a flowchart of a method of assessing an effect of a treatment 124 on an organ, e.g., a lung. The method may be performed by the system 100 of Figure 1 or the apparatus of Figure 8, described further below.

At block 702, a first measurement 108 for each of a plurality of regions 120 of a lung 122 is acquired. At block 704, a second measurement 110 for each of the plurality of regions 120 of the lung 122 is acquired, after acquisition of the first measurements and either after or during a delivery of the treatment 124 to the lung. In some embodiments, the second measurements 110 are acquired after delivery of the treatment 124 is complete. In other embodiments, the second measurements 110 may be acquired during delivery of the treatment 124 or partially during and partially after the treatment. The first measurements 108 and the second measurements 110 may be, for example, displacement measurements, velocity measurements, ventilation measurements, perfusion measurements, ventilation/perfusion (V/Q) ratio measurements, or any measurements that may be derived from any of the foregoing measurements.

The treatment 124 may be a non-uniform treatment characterized by varying levels of treatment delivery throughout the lung 122. The treatment 124 may be one or more of any therapy, including but not limited to radiation therapy, proton therapy, antibody therapy, surgery, valve placement, tissue ablation, or glue application. In one embodiment, the treatment 124 is a radiation therapy treatment that has associated regional treatment information 114 in the form of a dose map comprising a radiation level for each of the plurality of regions 120 of the lung 122.

The first measurement 108 for each of the plurality of regions 120 of the lung 122 and/or the second measurement 110 for each of a plurality of regions of the lung may be acquired by obtaining a time series or sequence of 2D images 126 of the lung, and processing the time series of 2D images to obtain a motion measurement for each of the plurality of regions. In one embodiment, obtaining a time series of 2D images 126 of the lung includes capturing a plurality of time series of 2D images of the lung, each from a different angle relative to the lung. The plurality of time series of 2D images 126 of the lung 122 may be captured from ten or fewer different angles. The plurality of time series of 2D images 126 may be captured asynchronously within the same breath, simultaneously, or during different breaths, or any combination thereof.

In one embodiment, processing the time series of 2D images 126 includes cross-correlating the 2D images of the lung 122. Processing the time series of 2D images 126 may also include reconstructing motion measurements for each of the plurality of regions 120 of the lung 122 from the time series of 2D images of the lung. To this end, reconstructing motion measurements may include reconstructing 3D motion measurements without first reconstructing a 3D image. Processing the time series of 2D images 126 may further include, for each of the plurality of regions 120 of the lung 122, deriving a volume measurement from one or more motion measurements associated with that region.

While the first measurements 108 and the second measurements 110 may be acquired in the same manner, it will be understood that these measurements may be acquired in different manners. For example, the first measurements 108 may be acquired using an x-ray imaging apparatus, while the second measurements 110 may be acquired using an MRI imaging apparatus. So long as the respective technique acquire the same type, e.g., ventilation, perfusion, etc., of first measurements 108 and the second measurements 110, the specific method of acquisition is not important.

At block 706, a regional change measurement 112 for each of the plurality of regions 120 of the lung 122 is obtained based on the first measurement 108 and the second measurement 110 of the region. A regional change measurement 112 for a region 120 may be obtained by comparing the first measurement 108 of the region to the second measurement 110 of the region. For example, a difference between the first measurement 108 of the region 120 and the second measurement 110 of the region may be determined.

At block 708, a treatment effect 116 is determined based the plurality of regional change measurements 112 and regional treatment information 114 of the treatment 124 delivered to the lung 122. A treatment effect 116 may be determined by mapping each of the plurality of regional change measurements 112 with a corresponding regional treatment information 114 of the treatment 124 delivered to the lung 122; and deriving the treatment effect from the mapping.

In one embodiment, the treatment effect 116 is derived from the mapping by fitting a line 504, 604 through a plot 500, 600 of regional change measurements 112 as a function of regional treatment information 114. Based on the treatment effect 116, it may be determined whether the treatment 124 has altered regional lung function. An assessment of lung function may also be made based on the treatment effect 116. The treatment effect 116 may be indicative of one of: a) no change in lung function; b) change in lung function linked to treatment; or c) change in lung function not linked to treatment.

In an optional embodiment, at block 710, prior to obtaining a regional change measurement 112 for each of the plurality of regions 120 of the lung 122 (block 706), the first measurements 108 and the second measurements 110 obtained in blocks 702 and 704 respectively, are associated with a fluid flow structure, e.g., an airway tree 138, of the lung 122. In other words, a first type, e.g., tissue motion, of the first measurements 108 and the second measurements 110 obtained in blocks 702 and 704 are associated with the airway tree 138 to create a second type, e.g., regional airway flow, of the first measurements 108 and the second measurements 110. Then in block 706, regional airway flow change measurements 112 are obtained based on the regional airway flow measurements.

In another optional embodiment, at block 712, prior to determining a treatment effect 116 (block 708), the plurality of regional change measurements 112 obtained in block 706 are associated with a fluid flow stmcture, e.g., an airway tree 138, of the lung 122. In other words, a first type, e.g., regional tissue motion change, of the regional change measurements 112 obtained in block 706 are associated with the airway tree 138 to create a second type, e.g., regional airway flow change, of the regional change measurements 112. Then in block 708, the regional airway flow change measurements are processed together with their corresponding regional treatment information 114 to determine a treatment effect.

Figure 8 is a schematic block diagram of an apparatus 800 for assessing an effect of a treatment 124 on an organ. The apparatus 800 may include one or more processors 802 configured to access and execute computer-executable instructions stored in at least one memory 804. The processor 802 may be implemented as appropriate in hardware, software, firmware, or combinations thereof.

The processor 802, implemented in hardware may be a general-purpose processor. A general-purpose processor may be a microprocessor, but in the alternative, the processor may be any conventional processor, controller, microcontroller, or state machine. The processor 802 may include, without limitation, a central processing unit (CPU), a digital signal processor (DSP), a reduced instruction set computer (RISC) processor, a complex instruction set computer (CISC) processor, a microprocessor, a microcontroller, a field programmable gate array (FPGA), a System-on-a-Chip (SOC), or other programmable logic, discrete gate or transistor logic, discrete hardware components, or any combination thereof, or any other suitable component designed to perform the functions described herein. The processor 802 may also include one or more application-specific integrated circuits (ASICs) or application-specific standard products (ASSPs) for handling specific data processing functions or tasks. The processor 802 may also be implemented as a combination of computing components, e.g., a combination of a DSP and a microprocessor, a plurality of microprocessors, one or more microprocessors in conjunction with a DSP, or any other such configuration.

Software or firmware implementations of the processor 802 may include computer-executable or machine-executable instructions written in any suitable programming language to perform the various functions described herein. Software shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise. The software may reside on a computer-readable medium. A computer-readable medium may include, by way of example, a smart card, a flash memory device (e.g., card, stick, key drive), random access memory (RAM), read only memory (ROM), programmable ROM (PROM), erasable PROM (EPROM), electrically erasable PROM (EEPROM), a general register, or any other suitable non-transitory medium for storing software.

The memory 804 may include, but is not limited to, random access memory (RAM), flash RAM, magnetic media storage, optical media storage, and so forth. The memory 804 may include volatile memory configured to store information when supplied with power and/or non-volatile memory configured to store information even when not supplied with power. The memory 804 may store various program modules, application programs, and so forth that may include computer-executable instructions that upon execution by the processor 802 may cause various operations to be performed. The memory 804 may further store a variety of data manipulated and/or generated during execution of computer-executable instructions by the processor 802.

The apparatus 800 may further include one or more interfaces 806 that may facilitate communication between the apparatus 800 and one or more other apparatuses using any suitable communications standard. Eor example, the interface 806 may enable the receipt of image datasets from an imaging apparatus 128, where the image datasets represent images 126 captured by the imaging apparatus. The interface 806 may also enable the receipt of regional treatment information 114 from a treatment apparatus 142. The interface 806 may be a LAN interface that implements protocols and/or algorithms that comply with various communication standards of the Institute of Electrical and Electronics Engineers (IEEE), such as IEEE 802.11, while a cellular network interface implement protocols and/or algorithms that comply with various communication standards of the Third Generation Partnership Project (3GPP) and 3GPP2, such as 3G and 4G (Long Term Evolution), and of the Next Generation Mobile Networks (NGMN) Alliance, such as 5G.

The memory 804 may store various program modules, application programs, and so forth that may include computer-executable instructions that upon execution by the processor 802 may cause various operations to be performed. Eor example, the memory 804 may include an operating system module (0/S) 808 that may be configured to manage hardware resources such as the network interface 806 and provide various services to applications executing on the apparatus 800.

The memory 804 stores additional program modules such as a measurement acquisition module 810, a measurement change module 812, a treatment effect module 814, a mapping module 816, and an airway tree module 818, each of which includes functions in the form of logic and rules that respectively support and enable the various functions described above with reference to Eigures 1 and 7, including: a) the acquiring of first measurements 108 and second measurements 110, b) the obtaining of regional change measurements 112, c) the determining of treatment effects 116, d) the associating of first measurements and second measurements with a fluid flow structure, e.g., airway tree, vascular tree, etc., prior to the obtaining of regional change measurements, and e) the associating of first measurements and second measurements with a fluid flow structure e.g., airway tree, vascular tree, etc., prior to the determining of treatment effects. Although illustrated as separate modules in Eigure 8, one or more of the modules may be a part of or a submodule of another module. Eor example, the mapping module 816 may be a submodule of the treatment effect module 814.

The modules 810, 812, 814, 816, 818 disclosed herein may be implemented in hardware, or software and/or firmware implementations executed on a hardware platform. The hardware may be the same as described above with respect to the processor 802. Likewise, the software and/or firmware implementations may be the same as described above with respect to the processor 802.

### Example Case Study 1 - Assessment of Radiation Treatment

A relationship between radiation exposure and the change in regional ventilation was explored. A regional dose distribution used for treatment planning was co-registered (e.g. mapped) to the CT used during calculation of the regional ventilation data, producing a dose contour map (see Figure 9A). This allowed direct comparison of ventilation measured at each location with the corresponding dose level. Furthermore, since XV ventilation fields at every time point were registered (e.g. mapped) to the same CT, the regional dose could also be compared to the regional change in normalized specific ventilation. In Figure 9B, the relationship is presented as three separate box-plots corresponding to dose levels of D < 0.1 Gy, 0.1 < D < 20 Gy, and D > 20 Gy. A positive value of normalized specific ventilation difference represents an increase in normalized specific ventilation compared to pretreatment, while a negative value denotes a decrease in normalized specific ventilation compared to pre-treatment.

For this patient, it is evident from Figure 9B that there is no clear relationship between dose and local changes in specific ventilation. This implies that this patient has not developed radiation pneumonitis.

### Example Case Study 2 - Assessment of Radiation Treatment

In this case, the change in normalized ventilation appears to be related to local dose (see Figure lOA). The box-plots in Figures lOB and IOC show a large spread in normalized specific ventilation difference. While at 4 months there was no relationship between dose and ventilation (Figure lOB), at 12 months there was a negative change in ventilation at 0.2 < D < 20 Gy and D > 20 Gy and a positive change at D < 0.1 Gy (Figure IOC). This finding, combined with the region of low specific ventilation in the right lung, could indicate the development of a radiation-induced disease such as pneumonitis.

## Claims

1. A system (100) for assessing an effect (116) of a treatment (124) on an organ (122) comprising:
a measurement acquisition module (102) configured to:
acquire a first measurement (108) for each of a plurality of regions (120) of the organ, and
acquire, after acquisition of the first measurements, a second measurement (110) for each of the plurality of regions (120) of the organ;
a measurement change module (104) configured to obtain a regional change measurement (112) for each of the plurality of regions (120) of the organ based on the first measurement and the second measurement of the region; and
a treatment effect module (106) configured to determine a treatment effect (116) based on the regional change measurements (112) of the plurality of regions (120) compared against corresponding regional treatment information (114) of the treatment (124) delivered to the organ (122);
**characterised in that** the treatment effect module (106) is configured to determine a treatment effect by mapping
each of the plurality of regional change measurements (112) with a corresponding regional treatment information (114) of the treatment (124) delivered to the organ to generate a mapping; and by deriving the treatment effect (116) from the mapping.

2. The system of claim 1, wherein the measurement acquisition module (102) acquires either of a first measurement (108) for each of a plurality of regions (120) of the organ or a second measurement (110) for each of a plurality of regions (120) of the organ by being configured to:
obtain a time series of two-dimensional (2D) images (126) of the organ; and
process the time series of 2D images (126) to obtain a motion measurement for each of the plurality of regions.

3. The system of claim 2, wherein the measurement acquisition module (102) obtains a time series of 2D images (126) of the organ (122) by being configured to capture a plurality of time series of 2D images (126) of the organ, each from a different angle relative to the organ; and
optionally
wherein the plurality of time series of 2D images of the organ are captured from no more than ten different angles; and/or
optionally
wherein the plurality of time series of 2D images (126) are captured simultaneously.

4. The system of claim 2, wherein the measurement acquisition module (102) processes the time series of 2D images (126) by being further configured to cross-correlate the 2D images of the organ; and
optionally
wherein the measurement acquisition module (102) processes the time series of 2D images (126) by being configured to reconstruct motion measurements for each of the plurality of regions of the organ from the time series of 2D images (126) of the organ; and
optionally
wherein the measurement acquisition module (102) reconstructs motion measurements by being configured to reconstruct 3D motion measurements without first reconstructing a 3D image; and
further optionally
wherein the measurement acquisition module (102) processes the time series of 2D images (126) by being configured to, for each of the plurality of regions (120) of the organ, derive a volume measurement from one or more motion measurements associated with that region.

5. The system of any one of claims 1 to 4, wherein the measurement acquisition module (102) is configured to:
acquire each of the plurality of first measurements (108) before the treatment, and each of the plurality of second measurements (110) is acquired either during the treatment (124) or after the treatment (124), or
acquire each of the plurality of first measurements (108) during the treatment (124), and each of the plurality of second measurements (110) is acquired after the treatment (124).

6. The system of any one of claims 1 to 5, wherein the first measurement (108) and the second measurement (110) are one of: displacement measurements, velocity measurements, ventilation measurements, perfusion measurements, ventilation/perfusion (V/Q) ratio measurements, or any measurements that may be derived from any of the foregoing measurements.

7. The system of any one of claims 1 to 6, wherein the treatment effect module (106) derives the treatment effect (116) from the mapping by being configured to fit a line (504, 604) through a plot (500, 600) of regional change measurements (112) as a function of regional treatment information (114).

8. The system of any one of claims 1 to 7, wherein the treatment effect (116) is indicative of one of:
a) no change in organ function;
b) change in organ function linked to treatment; or
c) change in organ function not linked to treatment.

9. The system of any one of claims 1 to 8, wherein the treatment (124) is a non-uniform treatment **characterized by** varying levels of treatment delivery throughout the organ; and
optionally
wherein the treatment (124) is a radiation therapy treatment, and the regional treatment information (114) is a dose map (202) comprising a radiation level for each of the plurality of regions (120) of the organ (122).

10. The system of any one of claims 1 to 9, wherein the measurement acquisition module (102) is further configured to either of:
associate the plurality of first measurements (108) and the plurality of second measurements (110) to a fluid flow structure of the organ, prior to obtaining a regional change measurement (112) for each of the plurality of regions (120) of the organ; or
associate the plurality of regional change measurements (112) to a fluid flow structure of the organ, prior to determining a treatment effect (116).

11. A non-transitory computer readable storage medium having a computer program stored therein, that when executed by a processor (802) of a computer, causes the computer to execute steps directed to assessing an effect (116) of a treatment (124) on an organ (122), the steps comprising:
acquiring a first measurement (108) for each of a plurality of regions (120) of the organ;
acquiring, after acquisition of the first measurements, a second measurement (110) for each of the plurality of regions (120) of the organ;
obtaining a regional change measurement (112) for each of the plurality of regions (120) of the organ based on the first measurement (108) and the second measurement (110) of the region (120); and
determining a treatment effect (116) based on the regional change measurements (112) of the plurality of regions (120) compared against corresponding regional treatment information (114) of the treatment (124) delivered to the organ;
**characterised in that** the treatment effect (116) is determined by:
mapping each of the plurality of regional change measurements (112) with a corresponding regional treatment information (114) of the treatment (124) delivered to the organ (122) to generate a mapping; and
deriving the treatment effect (116) from the mapping.

12. The non-transitory computer readable storage medium of claim 11, wherein:
each of the plurality of first measurements(108) is acquired before the treatment (124), and each of the plurality of second measurements (110) is acquired either during the treatment or after the treatment, or
each of the plurality of first measurements is acquired during the treatment, and each of the plurality of second measurements is acquired after the treatment.

13. The non-transitory computer readable storage medium of claims 11 or 12, wherein the first measurement (108) and the second measurement (110) are one of: displacement measurements, velocity measurements, ventilation measurements, perfusion measurements, ventilation/perfusion (V/Q) ratio measurements, or any measurements that may be derived from any of the foregoing measurements.

14. The non-transitory computer readable storage medium of any one of claims 11 to 13, wherein determining a treatment effect (116) comprises:
deriving the treatment effect (116) from the mapping comprises fitting a line (504, 604) through a plot (500, 600) of regional change measurements (112) as a function of regional treatment information (114); and
optionally
wherein the treatment effect is indicative of one of:
a) no change in organ function;
b) change in organ function linked to treatment; or
c) change in organ function not linked to treatment.

15. An apparatus (800) for assessing an effect (116) of a treatment (124) on an organ (122) comprising:
an interface (806);
a memory (804); and
a processor (802) coupled to the interface and the memory and configured to execute instructions in the memory to cause the apparatus to:
acquire a first measurement (108) for each of a plurality of regions (120) of the organ;
acquire, after acquisition of the first measurements, a second measurement (110) for each of the plurality of regions (120)of the organ;
obtain a regional change measurement (112) for each of the plurality of regions (120) of the organ based on the first measurement and the second measurement of the region; and
determine a treatment effect (116) based on the regional change measurements (112) of the plurality of regions compared against corresponding regional treatment information (114) of the treatment (124) delivered to the organ;
**characterised in that**
the processor (802) causes the apparatus (800) to determine a treatment effect (116) by being configured to execute instructions in the memory (804) to cause the apparatus to: receive regional treatment information (114) from a treatment apparatus (142) or treatment information source;
map each of the plurality of regional change measurements (112) with a corresponding regional treatment information (114) of the treatment (124) delivered to the organ (122) to generate a mapping; and
derive the treatment effect (116) from the mapping.

16. The apparatus of claim 15, wherein the processor (802) causes the apparatus (800) to derive the treatment effect (116) from the mapping by being configured to execute instructions in the memory (804) to cause the apparatus fit a line 504, 604) through a plot (500, 600) of regional change measurements (112) as a function of regional treatment information (114).

## Patentansprüche

1. System (100) zum Beurteilen einer Wirkung (116) einer Behandlung (124) auf ein Organ (122), umfassend:
ein Messungserfassungsmodul (102), das hierzu ausgelegt ist:
Erfassen einer ersten Messung (108) für jede von mehreren Regionen (120) des Organs und
Erfassen, nach Erfassung der ersten Messungen, einer zweiten Messung (110) für jede der mehreren Regionen (120) des Organs;
ein Messungsänderungsmodul (104), das dazu ausgelegt ist, eine regionale Änderungsmessung (112) für jede der mehreren Regionen (120) des Organs zu erhalten, die auf der ersten Messung und der zweiten Messung der Region basiert; und
ein Behandlungswirkungsmodul (106), das dazu ausgelegt ist, eine Behandlungswirkung (116) basierend auf den regionalen Änderungsmessungen (112) der mehreren Regionen (120) im Vergleich zu entsprechenden regionalen Behandlungsinformationen (114) der auf das Organ (122) angewendeten Behandlung (124) zu bestimmen;
**dadurch gekennzeichnet, dass** das Behandlungswirkungsmodul (106) dazu ausgelegt ist, eine Behandlungswirkung zu bestimmen, indem
jede der mehreren regionalen Änderungsmessungen (112) mit entsprechenden regionalen Behandlungsinformationen (114) der auf das Organ angewendeten Behandlung (124) kartiert wird, um eine Kartierung zu erzeugen; und
durch Ableiten der Behandlungswirkung (116) von der Kartierung.

2. System nach Anspruch 1, wobei das Messungserfassungsmodul (102) entweder eine erste Messung (108) für jede von mehreren Regionen (120) des Organs oder eine zweite Messung (110) für jede von mehreren Regionen (120) des Organs erfasst, indem es hierzu ausgelegt ist:
Erhalten einer Zeitreihe von zweidimensionalen (2D) Bildern (126) des Organs; und
Verarbeiten der Zeitreihen von 2D-Bildern (126), um eine Bewegungsmessung für jede der mehreren Regionen zu erhalten.

3. System nach Anspruch 2, wobei das Messungserfassungsmodul (102) eine Zeitreihe von 2D-Bildern (126) des Organs (122) erhält, indem es dazu ausgelegt ist, mehrere Zeitreihen von 2D-Bildern (126) des Organs zu erfassen, jede aus einem anderen Winkel relativ zu dem Organ; und
optional
wobei die mehreren Zeitreihen von 2D-Bildern des Organs aus nicht mehr als zehn unterschiedlichen Winkeln erfasst werden; und/oder
optional
wobei die mehreren Zeitreihen von 2D-Bildern (126) gleichzeitig erfasst werden.

4. System nach Anspruch 2, wobei das Messungserfassungsmodul (102) die Zeitreihen von 2D-Bildern (126) verarbeitet, indem es ferner dazu ausgelegt ist, die 2D-Bilder des Organs miteinander zu korrelieren; und
optional
wobei das Messungserfassungsmodul (102) die Zeitreihen von 2D-Bildern (126) verarbeitet, indem es dazu ausgelegt ist, Bewegungsmessungen für jede der mehreren Regionen des Organs anhand der Zeitreihen von 2D-Bildern (126) des Organs zu rekonstruieren; und
optional
wobei das Messungserfassungsmodul (102) Bewegungsmessungen rekonstruiert, indem es dazu ausgelegt ist, 3D-Bewegungsmessungen zu rekonstruieren, ohne zuerst ein 3D-Bild zu rekonstruieren; und
ferner optional
wobei das Messungserfassungsmodul (102) die Zeitreihen von 2D-Bildern (126) verarbeitet, indem es dazu ausgelegt ist, für jede der mehreren Regionen (120) des Organs eine Volumenmessung von einer oder mehreren Bewegungsmessungen abzuleiten, die mit dieser Region verknüpft sind.

5. System nach einem der Ansprüche 1 bis 4, wobei das Messungserfassungsmodul (102) hierzu ausgelegt ist:
Erfassen jeder der mehreren ersten Messungen (108) vor der Behandlung, und jede der mehreren zweiten Messungen (110) wird entweder während der Behandlung (124) oder nach der Behandlung (124) erfasst, oder
Erfassen jeder der mehreren ersten Messungen (108) während der Behandlung (124), und jede der mehreren zweiten Messungen (110) wird nach der Behandlung (124) erfasst.

6. System nach einem der Ansprüche 1 bis 5, wobei die erste Messung (108) und die zweite Messung (110) eines hiervon sind: Verschiebungsmessungen, Geschwindigkeitsmessungen, Ventilationsmessungen, Perfusionsmessungen, Messungen des Ventilation/Perfusion (V/Q)-Verhältnisses oder beliebige andere Messungen, die von einer der vorstehenden Messungen abgeleitet werden können.

7. System nach einem der Ansprüche 1 bis 6, wobei das Behandlungswirkungsmodul (106) die Behandlungswirkung (116) von der Kartierung ableitet, indem sie dazu ausgelegt ist, eine Linie (504, 604) durch einen Plot (500, 600) regionaler Änderungsmessungen (112) in Abhängigkeit von regionalen Behandlungsinformationen (114) zu ziehen.

8. System nach einem der Ansprüche 1 bis 7, wobei die Behandlungswirkung (116) eines hiervon angibt:
a) keine Änderung der Organfunktion;
b) Änderung der Organfunktion, an die Behandlung gebunden; oder
c) Änderung der Organfunktion, nicht an die Behandlung gebunden.

9. System nach einem der Ansprüche 1 bis 8, wobei die Behandlung (124) eine nicht-einheitliche Behandlung ist, **gekennzeichnet durch** variierende Niveaus der Behandlungsanwendung im gesamten Organ; und
optional
wobei die Behandlung (124) eine Strahlentherapiebehandlung ist und es sich bei den regionalen Behandlungsinformationen (114) um eine Dosiskarte (202) handelt, die ein Strahlungsniveau für jede der mehreren Regionen (120) des Organs (122) umfasst.

10. System nach einem der Ansprüche 1 bis 9, wobei das Messungserfassungsmodul (102) ferner zu einem hiervon ausgelegt ist:
Verknüpfen der mehreren ersten Messungen (108) und der mehreren zweiten Messungen (110) mit einer Fluidflussstruktur des Organs, bevor eine regionale Änderungsmessung (112) für jede der mehreren Regionen (120) des Organs erhalten wird; oder
Verknüpfen der mehreren regionalen Änderungsmessungen (112) mit einer Fluidflussstruktur des Organs, bevor eine Behandlungswirkung (116) bestimmt wird.

11. Nicht-transitorisches computerlesbares Datenspeichermedium, auf dem ein Computerprogramm gespeichert ist, das, wenn es von einem Prozessor (802) eines Computers ausgeführt wird, den Computer veranlasst, Schritte auszuführen, die darauf abzielen, eine Wirkung (116) einer Behandlung (124) auf ein Organ (122) zu beurteilen, wobei die Schritte umfassen:
Erfassen einer ersten Messung (108) für jede von mehreren Regionen (120) des Organs;
Erfassen, nach Erfassung der ersten Messungen, einer zweiten Messung (110) für jede der mehreren Regionen (120) des Organs;
Erhalten einer regionalen Änderungsmessung (112) für jede der mehreren Regionen (120) des Organs, die auf der ersten Messung (108) und der zweiten Messung (110) der Region (120) basiert; und
Bestimmen einer Behandlungswirkung (116) basierend auf den regionalen Änderungsmessungen (112) der mehreren Regionen (120) im Vergleich zu entsprechenden regionalen Behandlungsinformationen (114) der auf das Organ angewendeten Behandlung (124);
**dadurch gekennzeichnet, dass** die Behandlungswirkung (116) hierdurch bestimmt wird:
Kartieren jeder der mehreren regionalen Änderungsmessungen (112) mit entsprechenden regionalen Behandlungsinformationen (114) der auf das Organ (122) angewendeten Behandlung (124), um eine Kartierung zu erzeugen; und
Ableiten der Behandlungswirkung (116) von der Kartierung.

12. Nicht-transitorisches computerlesbares Datenspeichermedium nach Anspruch 11, wobei:
jede der mehreren ersten Messungen (108) vor der Behandlung (124) erfasst wird und jede der mehreren zweiten Messungen (110) entweder während der Behandlung oder nach der Behandlung erfasst wird, oder
jede der mehreren ersten Messungen während der Behandlung erfasst wird und jede der mehreren zweiten Messungen nach der Behandlung erfasst wird.

13. Nicht-transitorisches computerlesbares Datenspeichermedium nach Anspruch 11 oder 12, wobei die erste Messung (108) und die zweite Messung (110) eines hiervon sind: Verschiebungsmessungen, Geschwindigkeitsmessungen, Ventilationsmessungen, Perfusionsmessungen, Messungen des Ventilation/Perfusion (V/Q)-Verhältnisses oder beliebige andere Messungen, die von einer der vorstehenden Messungen abgeleitet werden können.

14. Nicht-transitorisches computerlesbares Datenspeichermedium nach einem der Ansprüche 11 bis 13, wobei das Bestimmen einer Behandlungswirkung (116) umfasst:
das Ableiten der Behandlungswirkung (116) von der Kartierung umfasst das Ziehen einer Linie (504, 604) durch einen Plot (500, 600) regionaler Änderungsmessungen (112) in Abhängigkeit von regionalen Behandlungsinformationen (114); und
optional
wobei die Behandlungswirkung eines hiervon angibt:
a) keine Änderung der Organfunktion;
b) Änderung der Organfunktion, an die Behandlung gebunden; oder
c) Änderung der Organfunktion, nicht an die Behandlung gebunden.

15. Vorrichtung (800) zum Beurteilen einer Wirkung (116) einer Behandlung (124) auf ein Organ (122), umfassend:
eine Schnittstelle (806);
einen Speicher (804); und
einen Prozessor (802), der an die Schnittstelle und den Speicher gekoppelt ist und dazu ausgelegt ist, Anweisungen im Speicher auszuführen, um die Vorrichtung hierzu zu veranlassen:
Erfassen einer ersten Messung (108) für jede von mehreren Regionen (120) des Organs;
Erfassen, nach Erfassung der ersten Messungen, einer zweiten Messung (110) für jede der mehreren Regionen (120) des Organs;
Erhalten einer regionalen Änderungsmessung (112) für jede der mehreren Regionen (120) des Organs, die auf der ersten Messung und der zweiten Messung der Region basiert; und
Bestimmen einer Behandlungswirkung (116) basierend auf den regionalen Änderungsmessungen (112) der mehreren Regionen im Vergleich zu entsprechenden regionalen Behandlungsinformationen (114) der auf das Organ angewendeten Behandlung (124);
**dadurch gekennzeichnet, dass**
der Prozessor (802) die Vorrichtung (800) dazu veranlasst, eine Behandlungswirkung (116) zu bestimmen, indem sie dazu ausgelegt ist, Anweisungen im Speicher (804) auszuführen, um die Vorrichtung hierzu zu veranlassen: Empfangen regionaler Behandlungsinformationen (114) von einer Behandlungsvorrichtung (142) oder einer Behandlungsinformationsquelle;
Kartieren jeder der mehreren regionalen Änderungsmessungen (112) mit entsprechenden regionalen Behandlungsinformationen (114) der auf das Organ (122) angewendeten Behandlung (124), um eine Kartierung zu erzeugen; und
Ableiten der Behandlungswirkung (116) von der Kartierung.

16. Vorrichtung nach Anspruch 15, wobei der Prozessor (802) die Vorrichtung (800) dazu veranlasst, die Behandlungswirkung (116) von der Kartierung abzuleiten, indem er dazu ausgelegt ist, Anweisungen im Speicher (804) auszuführen, um die Vorrichtung dazu zu veranlassen, eine Linie (504, 604) durch einen Plot (500, 600) regionaler Änderungsmessungen (112) in Abhängigkeit von regionalen Behandlungsinformationen (114) zu ziehen.

## Revendications

1. Système (100) permettant d'évaluer un effet (116) d'un traitement (124) sur un organe (122) comprenant :
un module d'acquisition de mesures (102) configuré pour :
acquérir une première mesure (108) pour chaque région d'une pluralité de régions (120) de l'organe, et
acquérir, après l'acquisition des premières mesures, une seconde mesure (110) pour chaque région de la pluralité de régions (120) de l'organe ;
un module de changement de mesure (104) configuré pour obtenir une mesure de changement régional (112) pour chaque région de la pluralité de régions (120) de l'organe sur la base de la première mesure et de la seconde mesure de la région ; et
un module d'effet de traitement (106) configuré pour déterminer un effet de traitement (116) sur la base des mesures de changement régional (112) de la pluralité de régions (120) par comparaison avec des informations de traitement régional (114) correspondantes du traitement (124) administré à l'organe (122) ;
**caractérisé en ce que** le module d'effet de traitement (106) est configuré pour déterminer un effet de traitement par mise en correspondance
de chaque mesure de la pluralité de mesures de changement régional (112) avec une information de traitement régional (114) correspondante du traitement (124) administré à l'organe pour créer une mise en correspondance ; et
par dérivation de l'effet de traitement (116) à partir de la mise en correspondance.

2. Système selon la revendication 1, dans lequel le module d'acquisition de mesure (102) acquiert soit une première mesure (108) pour chaque région d'une pluralité de régions (120) de l'organe, soit une seconde mesure (110) pour chaque région d'une pluralité de régions (120) de l'organe en étant configuré pour :
obtenir une série chronologique d'images bidimensionnelles (2D) (126) de l'organe ; et
traiter la série chronologique d'images 2D (126) pour obtenir une mesure de mouvement pour chaque région de la pluralité de régions.

3. Système selon la revendication 2, dans lequel le module d'acquisition de mesure (102) obtient une série chronologique d'images 2D (126) de l'organe (122) en étant configuré pour capturer une pluralité de séries chronologiques d'images 2D (126) de l'organe, chacune à partir d'un angle différent par rapport à l'organe ; et facultativement
dans lequel la pluralité de séries chronologiques d'images 2D de l'organe est capturée à partir d'un maximum de dix angles différents ; et/ou
facultativement
dans lequel la pluralité de séries chronologiques d'images 2D (126) est capturée simultanément.

4. Système selon la revendication 2, dans lequel le module d'acquisition de mesure (102) traite la série chronologique d'images 2D (126) en étant en outre configuré pour corréler de manière croisée les images 2D de l'organe ; et
facultativement
dans lequel le module d'acquisition de mesures (102) traite la série chronologique d'images 2D (126) en étant configuré pour reconstruire des mesures de mouvement pour chaque région de la pluralité de régions de l'organe à partir de la série chronologique d'images 2D (126) de l'organe ; et
facultativement
dans lequel le module d'acquisition de mesures (102) reconstruit des mesures de mouvement en étant configuré pour reconstruire des mesures de mouvement 3D sans reconstruire au préalable une image 3D ; et
facultativement en outre
dans lequel le module d'acquisition de mesure (102) traite la série chronologique d'images 2D (126) en étant configuré pour, pour chaque région de la pluralité de régions (120) de l'organe, dériver une mesure de volume à partir d'une ou de plusieurs mesures de mouvement associées à cette région.

5. Système selon l'une quelconque des revendications 1 à 4, dans lequel le module d'acquisition de mesure (102) est configuré pour :
acquérir chaque première mesure de la pluralité de premières mesures (108) avant le traitement, et chaque seconde mesure de la pluralité de secondes mesures (110) est acquise soit pendant le traitement (124), soit après le traitement (124), ou
acquérir chaque première mesure de la pluralité de premières mesures (108) pendant le traitement (124), et chaque seconde mesure de la pluralité de secondes mesures (110) est acquise après le traitement (124).

6. Système selon l'une quelconque des revendications 1 à 5, dans lequel la première mesure (108) et la seconde mesure (110) sont l'une parmi : mesures de déplacement, mesures de vitesse, mesures de ventilation, mesures de perfusion, mesures de rapport ventilation/perfusion (V/Q), ou de quelconques mesures qui peuvent être dérivées d'une quelconque des mesures précédentes.

7. Système selon l'une quelconque des revendications 1 à 6, dans lequel le module d'effet de traitement (106) dérive l'effet de traitement (116) à partir de la mise en correspondance en étant configuré pour s'ajuster à une ligne (504, 604) à travers un tracé (500, 600) de mesures de changement régional (112) en fonction d'informations de traitement régional (114).

8. Système selon l'une quelconque des revendications 1 à 7, dans lequel l'effet de traitement (116) est révélateur de l'un parmi :
a) aucun changement de la fonction de l'organe ;
b) changement de la fonction de l'organe lié au traitement ; ou
c) changement de la fonction de l'organe non lié au traitement.

9. Système selon l'une quelconque des revendications 1 à 8, dans lequel le traitement (124) est un traitement non uniforme **caractérisé par** des niveaux variables d'administration de traitement dans tout l'organe ; et
facultativement
dans lequel le traitement (124) est un traitement de radiothérapie, et les informations de traitement régional (114) sont une carte de doses (202) comprenant un niveau de rayonnement pour chaque région de la pluralité de régions (120) de l'organe (122).

10. Système selon l'une quelconque des revendications 1 à 9, dans lequel le module d'acquisition de mesure (102) est en outre configuré pour :
associer la pluralité de premières mesures (108) et la pluralité de secondes mesures (110) à une structure d'écoulement de fluide de l'organe, avant d'obtenir une mesure de changement régional (112) pour chaque région de la pluralité de régions (120) de l'organe ; ou
associer la pluralité de mesures de changement régional (112) à une structure d'écoulement de fluide de l'organe, avant de déterminer un effet de traitement (116).

11. Support de stockage non transitoire lisible par ordinateur dans lequel est stocké un programme informatique qui, lorsqu'il est exécuté par un processeur (802) d'un ordinateur, amène l'ordinateur à exécuter des étapes visant à évaluer un effet (116) d'un traitement (124) sur un organe (122), les étapes comprenant :
l'acquisition d'une première mesure (108) pour chaque région d'une pluralité de régions (120) de l'organe ;
l'acquisition, après l'acquisition des premières mesures, d'une seconde mesure (110) pour chaque région de la pluralité de régions (120) de l'organe ;
l'obtention d'une mesure de changement régional (112) pour chaque région de la pluralité de régions (120) de l'organe sur la base de la première mesure (108) et de la seconde mesure (110) de la région (120) ; et
la détermination d'un effet de traitement (116) sur la base des mesures de changement régional (112) de la pluralité de régions (120) par comparaison avec des informations de traitement régional (114) correspondantes du traitement (124) administré à l'organe ;
**caractérisé en ce que** l'effet de traitement (116) est déterminé par :
la mise en correspondance de chaque mesure de la pluralité de mesures de changement régional (112) avec une information de traitement régional (114) correspondante du traitement (124) administré à l'organe (122) pour créer une mise en correspondance ; et
la dérivation de l'effet de traitement (116) à partir de la mise en correspondance.

12. Support de stockage non transitoire lisible par ordinateur selon la revendication 11, dans lequel :
chaque première mesure de la pluralité de premières mesures (108) est acquise avant le traitement (124), et chaque seconde mesure de la pluralité de secondes mesures (110) est acquise soit pendant le traitement, soit après le traitement, ou
chaque première mesure de la pluralité de premières mesures est acquise pendant le traitement, et chaque seconde mesure de la pluralité de secondes mesures est acquise après le traitement.

13. Support de stockage non transitoire lisible par ordinateur selon les revendications 11 ou 12, dans lequel la première mesure (108) et la seconde mesure (110) sont l'une parmi : mesures de déplacement, mesures de vitesse, mesures de ventilation, mesures de perfusion, mesures de rapport ventilation/perfusion (V/Q), ou de quelconques mesures qui peuvent être dérivées d'une quelconque des mesures précédentes.

14. Support de stockage non transitoire lisible par ordinateur selon l'une quelconque des revendications 11 à 13, dans lequel la détermination d'un effet de traitement (116) comprend :
la dérivation de l'effet de traitement (116) à partir de la mise en correspondance comprend l'ajustement d'une ligne (504, 604) à travers un tracé (500, 600) de mesures de changement régional (112) en fonction d'informations de traitement régional (114) ; et
facultativement
dans lequel l'effet de traitement est révélateur de l'un parmi :
a) aucun changement de la fonction de l'organe ;
b) changement de la fonction de l'organe lié au traitement ; ou
c) changement de la fonction de l'organe non lié au traitement.

15. Système (800) permettant d'évaluer un effet (116) d'un traitement (124) sur un organe (122) comprenant :
une interface (806) ;
une mémoire (804) ; et
un processeur (802) couplé à l'interface et à la mémoire est configuré pour exécuter des instructions dans la mémoire pour amener l'appareil à :
acquérir une première mesure (108) pour chaque région d'une pluralité de régions (120) de l'organe ;
acquérir, après l'acquisition des premières mesures, une seconde mesure (110) pour chaque région de la pluralité de régions (120) de l'organe ;
obtenir une mesure de changement régional (112) pour chaque région de la pluralité de régions (120) de l'organe sur la base de la première mesure et de la seconde mesure de la région ; et
déterminer un effet de traitement (116) sur la base des mesures de changement régional (112) de la pluralité de régions par comparaison avec des informations de traitement régional (114) correspondantes du traitement (124) administré à l'organe ;
**caractérisé en ce que**
le processeur (802) amène l'appareil (800) à déterminer un effet de traitement (116) en étant configuré pour exécuter des instructions dans la mémoire (804) pour amener l'appareil à : recevoir des informations de traitement régional (114) d'un appareil de traitement (142) ou d'une source d'informations de traitement ;
mettre en correspondance chaque mesure de la pluralité de mesures de changement régional (112) avec une information de traitement régional (114) correspondante du traitement (124) administré à l'organe (122) pour créer une mise en correspondance ; et
dériver l'effet de traitement (116) de la mise en correspondance.

16. Appareil selon la revendication 15, dans lequel le processeur (802) amène l'appareil (800) à dériver l'effet de traitement (116) de la mise en correspondance en étant configuré pour exécuter des instructions dans la mémoire (804) pour amener l'appareil à s'ajuster à une ligne (504, 604) à travers un tracé (500, 600) de mesures de changement régional (112) en fonction d'informations de traitement régional (114).
